Europäisches Patentamt

**European Patent Office**

Office européen des brevets

(19)

(11) Publication number: **0 199 141**
A2

(12) # EUROPEAN PATENT APPLICATION

(21) Application number: **86104321.4**

(22) Date of filing: **27.03.86**

(51) Int. Cl.⁴: **C 07 K 15/00**
C 12 P 21/00, C 12 N 5/00
C 12 N 15/00, G 01 N 33/577
G 01 N 33/573
//(C12P21/00, C12R1:91)

(30) Priority: **19.04.85 US 724991**

(43) Date of publication of application:
**29.10.86 Bulletin 86/44**

(84) Designated Contracting States:
**DE GB**

(71) Applicant: **Sloan-Kettering Institute For Cancer
Research
1275 York Avenue
New York New York 10021(US)**

(72) Inventor: **Lloyd, Kenneth O.
4525 Henry Hudson Parkway West
Bronx New York 10021(US)**

(72) Inventor: **Yin, Beatrice
136-76 72nd Avenue
Flushing New York 11367(US)**

(72) Inventor: **Sakamoto, Junichi
404 Minamigaoka Iris 1-10-62 Minamigaoka
Chikusaku Nagoya(JP)**

(72) Inventor: **Watanabe, Tadashi
Fujigaoki-162 Kodan 4-401
Meito-ku Nagoya 465(JP)**

(72) Inventor: **Furukawa, Koichi
524 East 84th Street
New York New York 10028(US)**

(72) Inventor: **Old, Lloyd J.
600 West End Avenue
New York New York 10024(US)**

(74) Representative: **Patentanwälte Schulze Horn und
Hoffmeister
Goldstrasse 36
D-4400 Münster(DE)**

(54) Monoclonal antibodies to human gastrointestinal cancer.

(57) Diagnostic panels for human gastrointestinal abnormalities such as cancer using mouse monoclonal antibodies are disclosed. These panels can be used in diagnosis and in therapeutic applications such as colon cancer.

EP 0 199 141 A2

0199141

This invention was partially made with United States government support under CA 08748 awarded by the National Cancer Institute. The government has certain rights in this invention.

## Background

This invention concerns monoclonal antibodies recognizing human gastrointestinal (GI) cells. The monoclonal antibodies recognize antigenic markers on normal as well as cancerous GI cells. Capable of distinguishing among normal GI cells as well as colon carcinomas, these mAbs are useful in diagnosis and prognosis of colon and gastrointestinal cancer. Examination of human GI specimens: tissues wastes, exudates, and fluids with these mAbs is a diagnostic procedure to probe for cancer of the gastrointestinal tract and especially colon cancer. These mAbs are of special importance because of the widespread occurrence of colon and stomach cancer.

In 1975 Köhler and Millstein introduced a procedure for the production of monoclonal antibodies (mAbs) using hybrid cells (hybridomas) which allows the production of almost unlimited quantities of antibodies of precise and reproducible specificity. Conventional antisera, produced by immunizing animals with tumor cells or other antigens, contain a myriad of different antibodies differing in their specificity and properties, whereas hybridomas produce a single antibody with uniform characteristics. The Kohler-Millstein procedure entails the fusion of spleen cells from an immunized animal with an immortal myeloma cell line. From the fused cells (hybridomas), clones are selected that produce antibody of the desired specificity. Each clone continues to produce only that one antibody. As hybridoma cells can be cultured indefinitely (or stored frozen in liquid nitrogen), a constant supply of antibody is assured.

Antibodies are proteins that have the ability to combine with and recognize other molecules, known as antigens. Monoclonal antibodies are no different from other antibodies except that they are very uniform in their properties and recognize only one antigen or a portion of an antigen known as a determinant.

In the case of cells, the determinant recognized is an antigen on or in the cell which reacts with the antibody. It is through these cell antigens that a particular antibody recognizes, i.e. reacts with, a particular kind of cell. Thus the cell antigens are markers by which the cell is identified.

These antigenic markers may be used to observe the normal process of cell differentiation and to locate abnormalities within a given cell system. The process of differentiation is accompanied by changes in the cell surface antigenic phenotype, and antigens that distinguish cells belonging to distinct differentiation lineages or distinguish cells at different phases in the same differentiation lineage may be observed if the correct antibody is available. Initial recognition of differentiation antigens came about through analysis of surface antigens of T-cell leukemias of the mouse and the description of the TL, Thy-1, and Lyt series of antigens. (Old, Lloyd J., Cancer Research, 41, 361-375, February 1981) The analysis of these T-cell differentiation antigens was greatly simplified by the availability of normal T cells and B cells of mouse and man and is relatively advanced. (See Patents #4,361,549-550; #4,364,932-37 and #4,363,799 concerning mAb to Human T-cell antigens). There is further experimentation to be done concerning differentiation antigens displayed on normal and neoplastic cells belonging to other lineages.

The preparation of hybrid cell lines can be successful or not depending on such experimental factors as nature of the innoculant, cell growth conditions, hybridization conditions etc. Thus it is not always possible to predict successful hybridoma preparation with one cell line although success may have been achieved with another cell line.

Progress in defining surface antigens on melanocytes was made possible by the recently discovered technique of culturing melanocytes from normal skin (Eisinger, et al., Proc. Nat'l. Acad. Sci. USA, 79 2018 (March 1982). This method provides a renewable source of proliferating cells for the analysis of melanocyte differentiation antigens. Likewise, a large number of cell lines derived from melanomas have now been established and these have facilitated the analysis of melanoma surface antigens. The advent of mAbs has greatly accelerated knowledge about the surface antigens of malignant melanoma. Cell markers on both melanomas and melanocytes have been identified. A panel of typing monoclonal antibodies has been selected which recognizes differentiation antigen characteristics at each stage of development in both melanocytes and melanomas. These differentiation antigens may be used to classify melanocytes and melanomas and to group them into characteristic sub-sets. Dippold et al. Proc. Nat'l. Acad. Sci. U.S.A. 77, 6114 (1980) and Houghton, et al. J. Exp. Med. 156, 1755 (1982). Immunoassay of melanocytes and melanoma cells within sub-sets is thus made possible.

## Summary

Cancers of the gastrointestinal tract are especially widespread; stomach cancer in Japan, colon cancer in the west and U.S.A. Early diagnosis would be desireable to prevent loss of life and prescribe alternatives to drastic surgery. Positive diagnosis can help to support the surgical decision. Cytohistological methods to date are not always successful. A panel group of mAbs of the present invention recognizing cancerous GI cells enables such a distinction. In addition, the panel distinguishes normal from cancerous cells.

The invention thus comprises hybridoma cell lines producing mAbs recognizing human colon cancer cells, from the group of AS33, AS37, CLK314, CLH70, HT29/15, HT29/26, CLT307, CLT86, V-215, V-715. A preferred group comprises AS33, AS37, CLH70, CLK314, CLT86, CLT307, HT29-15, and HT29-26. These mAbs of the invention recognize colon or GI glycoprotein (gp) antigens molecular weights 25kd, 29kd and 95kd (mAbs CLH70, HT29/26 and CLT479 respectively). mAb CLT152 recognizes a protein antigen of 52 kd. The antigens for CLH6, CLT85, CLT174 and HT29/36 are heat stable and therefore probably glycolipids. CLT85, CLT479, CLT174, HT29/36, CLH68, CLT152 and HT29/15 are gamma sub one

(gamma$_1$) immunoglobulins. HT29/26 is a gamma sub 2A (gamma$_{2A}$) immunoglobulin. HT29/36 is a gamma sub 3 (gamma$_3$) immunoglobulin and CLT218, CLT307, CLT86 and CLH6 are mu immunoglobulins. (HT 29/36 is the same mAb as HT 29-36 or 29/36. HT 29/15 is the same mAb as HT 29-15 or 29/15 and HT 29/26 is the same mAb as HT 29-26 or 29/26. As 33 is the same monoclonal antibody as A-33 and AS37 is the same as A-37. In the tables below, CLH6 is the same mAb as 6, CLT86 is the same as 86, CLT85 is the same as 85, CLT307 is the same as 307, CLT479 is the same as 479, CLT174 is the same as 174, CLH 70 is the same as 70, CLT 15 is the same as 15, CLH70 is the same as 70, CLT152 is the same as 152. The following hybridoma cell lines and monoclonal antibodies produced therefrom namely: HT 29/15, HT 29/26, HT 29/36, CLH 6 (or 6), CLT 85 (or 85), CLT 479 (or 479), CLT 174 (or 174), CLH68 (or 68), CLT 152 (or 152), CLH 70 (or 70) CLT 218 (or 218), CLT 15 (or 15), CLT 307 (or 307) and CLT 86 (or 86) have been disclosed and claimed in a previously filed, co-pending application filed March 11, 1983, Serial No. 474,415 herein incorporated by reference. These are described in a publication Sakamoto et al _____ herein incorporated by reference.

## Description

A preferred embodiment of the present invention is to test a human specimen as for example human body tissues, wastes, fluids and exudates with each of the monoclonal antibodies of the panel. The cells are tested or contacted separately with each of the monoclonal antibodies in a series of dilutions. Thus, an assay for cancer is possible with minimal patent disruption. Indeed, the present invention permits testing of human GI waste specimens for cell fragments containing antigenic markers for the monoclonal antibodies. Entire cells need not be present. Cytohistological methods requiring whole cells are not always successful.

The monoclonal antibodies of the present invention were prepared by the Kohler-Millstein procedure wherein spleen cells from a mouse (or other mammal) immunized with human cancerous colon cells or pancreas from established human tumor cell lines of fresh tumor tissue were fused with mouse myeloma to form hybridomas. By serological screening, antibodies from these hybridomas were found which recognize differentiation antigens on normal bladder and/or cancerous bladder. Other tissues, both normal and cancerous, may be recognized as well by some of these monoclonal antibodies. A system of classification of normal as well as cancerous colon based on these differentiation antigens is now possible, and serological assays for tumors of the colon have been developed. These assays are of special use in the early diagnosis of gastrointestinal cancer especially colon cancer.

TISSUE CULTURE:

Cultured human colon cancer cell lines came from Leibowitz and from the collection of J. Fogh at Sloan-Kettering Institute. Cultures of other established human cell lines and normal tissue cells have been described.

PRODUCTION OF MOUSE MONOCLONAL ANTIBODIES

BALB/c mice were immunized with either a colon carcinoma or pancreas carcinoma cell line or with fresh colon cancer tissues. Subcutaneous and intraperitoneal injections of 1x10 cells were given three to ten times at intervals of 2 weeks. Three days after the last injection, the fusion of immune spleen cells with mouse myeloma MOPC-21 NS/1 cells was performed as described. Culture supernatents were tested for antibody by the anti-mouse IG mixed hemmaglutination assay (MHA) or Protein A assay(PA) on a panel of cultured cell lines of colon and other types of tissue cells. After subcloning five to six times, hybridoma cells were injected subcutaneously into nu/nu mice (Swiss background) and sera from mice with tumors were collected and used for serological, immunopathological and biochemical characterization. In general these methods have been described in Ueda et al. (1981) Proc. Natl. Acad. Sci. U.S.A. 78:5122, Dippold et al. (1980) Proc. Natl. Acad. Sci. U.S.A. 77:6114.

SEROLOGICAL PROCEDURES:

The MHA, on cultured cells using rabbit anti-mouse Ig and mouse anti-SRBC has been described. Absorption tests, assessment of heat stability and proteinase sensitivity and antibody subclass determination were also performed as described. See Dippold et al, _Supra_ and Ueda et al. _Supra_, Pfreundschuh et al. (1978) Proc. Natl. Acad. Sci. USA 75:5122, Ueda et al. (1979) J. Exp. Med. 150:564

IMMUNOPATHOLOGICAL PROCEDURES

Immunofluorescent staining of cryostat sections with fluorescein conjugated goat anti-mouse Ig (Cappel Laboratories) was performed as described. (Fradet et al. (1984) Proc. Natl. Acad. Sci USA January _____. Immunoperoxidase staining, using monoclonal antibody, peroxidase conjugated goat anti-mouse Ig and 3-amino-9-ethylcarbazol (AEC) (Histoset, Ortho Diagnostic system) was carried out following procedures recommended by the manufacturer.

IMMUNOPRECIPITATION PROCEDURES:

Antibodies were tested for immunoprecipitation activity by using detergent solubilized cell extracts labeled by [³H] glucosamine. Nonidet P-40 solubilization of cells and immunoprecipitation procedures using Staphylococcus aureus have been described. Aliquots of 2X10 [³H] cpm from unfractionated cell extracts were used. Precipitated molecules were extracted with 60 1 of 0.01M Tris-Hcl PH 7.2/2.0% NaDodSO$_4$/ 12.0mg of dithiothreitol per ml/15% (weight/volume) sucrose/0.01% pyronin Y by heating 5 min at 100°C and were analysed by polyacrylamide gel electrophoresis. Dippold et al. _Supra_. Cairncross et al., (1962) Proc. Natl. Acad. Sci. USA _79_:5641.

The assay of the present invention comprises contacting a tissue containing colon cells with the antibody recognizing colon cell antigens, preferably monoclonal antibodies to one or more cell antigens of the gastrointestinal antigenic system, and observing the immunoserological or immunopathological antigenic reaction between said monoclonal antibody and said antigen. In a preferred embodiment of the invention, the tissue sample to be contacted is gastrointestinal tissue and the antigenic reaction of the contacted tissue is observed by well known techniques such as immunofluorescence, Rosette formation with sheep or human red blood cells linked to Protein A or anti-Ig direct absorption and the like. In another embodiment of the present invention, the tissue to be assayed is first excised and is then either freshly, or after being frozen or embedded in paraffin by methods well-known in the art, contacted with the monoclonal antibodies of the invention.  Observation of the reaction is as before.

In another preferred embodiment of the present invention, the tissue to be assayed comprises the intact body of an individual or a whole portion thereof.  The antibody, tagged with a radioactive or other energy-producing element, is administered to the individual, and the whole body or part thereof is scanned externally for localization of radioactivity at the site of cancerous gastrointestinal cells.  In another preferred embodiment cancerous colon cells are located.

The present invention also makes possible the treatment of gastrointestinal tumors in a patient wherein the monoclonal antibody recognizing the cell antigen of cancerous colon or other cancerous GI cells, is administered to the patient in an amount effective to inhibit the growth or proliferation of cancer cells.  In a preferred embodiment of this method, the antibody is tagged with a potentially tissue destructive agent which causes destruction of the cancer cells.  Examples of tissue destructive agents comprise chemotoxic agents, chemotherapeutic agents including vaccines, radionuclides, toxins, complement activators, clotting activators and the like. These examples are for illustrative purposes only and are not meant to limit the scope of the invention.

The following examples are intended to illustrate the invention without limiting same in any manner especially with respect to substantially functional equivalents of hybridomas, monoclonal antibodies and cell lines described and claimed herein.

The monoclonal antibodies selected for use in the present invention were derived from spleen cells of mice immunized with whole cells of colon carcinoma cell lines such as Tallevi and HT-29 fresh tumor cell lines or pancreatic tumor cell lines by fusion methods well known in the art.

A group of monoclonal antibodies which were found to recognize specific cell antigens of gastrointestinal cells, was selected as the gastrointestinal panel. This panel and other mAbs and the antigenic systems recognized are given in Tables I, II, III and IV. Heterogeneity of human colon carcinoma is therein noted. The table data point out and define the heterogeneity of colon carcinomas.

Gastrointestinal antigenic systems are defined by these mAbs as determined by serological analysis with over 70 tumor cell lines; 18 colon cancers, over 50 non-gastrointestinal cancers as well as immunopathology on frozen sections of normal adult and normal fetal tissue and cancer tissue. (See Table I, II, III and IV Reactivity with tissue of cancer patients is shown in Table V)

Eight monoclonal antibodies to cell surface antigens of human colon carcinoma were obtained by immunization with cultured human colon and pancreas carcinomas or with lysates of colon cancer cells. The distribution of the antigens detected was analysed on 164 normal and malignant cell lines (Table III) and on frozen sections of normal adult and fetal tissues (Table IV). Fifty five colon carcinomas and normal colonic tissue from the same patient were also examined (Table V).

One very restricted antigen, V-215 (gp140), were detected only on colon and four other cancer cell lines (Table III). In several patients, the antigen were expressed only on colon cancers but not in normal adjacent colon tissues (Table V).

A-33 antigen was found only on colonic and pancreatic cancer cell lines, and not in any normal adult tissues, it was present on both tumor and normal adjacent colonic tissues (Tables III, IV and V).

K-314 antigen (gp170) was only on colon and a few lung cancer cell lines (Tables III). In immunopathology, the antigen was not found in any normal adult tissues except some part of the proximal tubules of the kidney (Table IV).

A-37 antigen was on colon, some renal and hematopoietic cell lines but was found only in the proximal tubules of kidney in immunopathology staining (Tables III and IV).

HT-29-15 antigen (H-15) (Tables I-IV) was detected on colon, breast and lung cancer cell lines and also, in certain patients, was expressed on colon cancer tissues but not on their normal counterparts (Table V). H-15 determinants are carried on a high molecular weight glycoprotein and are neuraminidase-sensitive.

V-715 antigen (gp120) was expressed on colon, lung and renal cancers (Tables III and IV). V-715 antigen has a similar serological, and immunopathological characteristics with the Adenosine deaminase protein. H-70 (Table I-IV) antigen (gp29) was expressed on colon, lung, renal cancer and neuroblastoma cell lines.

HT-29-26 antigen (gp31) (H-26) was detected on almost all the epithelial tissues, but not in other tissues (Tables I-IV).

None of the antigens were related to A, B, H, I or Lewis blood group specificities.

## Example I

Several of the antigens, as defined by the monoclonal antibodies of the panel, are expressed differentially by cell populations within the adult GI system. CLT152 antigen is expressed by epithelial cells of the GI mucosa of esophagus, stomach, small intestine and colon, but is not found in other adult tissues. CLH70, CLT307, CLT86 and CLH68 antigens are expressed by adult stomach, small intestine and colon. CLT218 is expressed by adult small intestine and colon. HT29/26 is expressed by colon and some cells of small intestine in the adult. CLT15 also is expressed by normal colon epithelium as well as some upper GI cells except stomach in adult tissues. Thus the mAbs antigens HT29/26, CLT15, CLT218, CLH70, CLT307, CLT86 and CLH68 occur in adult colon epithelial cells; they vary among themselves in their pattern of distribution within the rest of the GI tract. There is some limited expression of these antigens in epithelial cells of other tissues as well [See

0199141

Table II]. Thus, for example, CLT218, CLT86, HT29/26 antigens are expressed on bronchial epithelium whereas CLH6, HT29/36 and HT29/15 are not. Thus, the panel antibodies differ in their expression on normal cells even as to similar cells of other tissues.

It is important that the mAbs CLH6, CLT85, and 29/36 do not react with normal adult tissue in immunopathology of frozen tissue sections but do react with distinct subsets of colon adenocarcinomas.

Serologically CLT85 reacts with approximately 11 of 17 colon cancer lines, and CLH6 with approximately 8 out of 17 colon cancer cell lines. CLT85 and CLH6 show no reaction with normal adult cells in serology.

From 23 further Köhler-Milstein fusions done as above of NS/1 myeloma with spleen cells, 8 more antibody producing clones were selected for further detailed analysis as discussed below. The serological specificities of these antibodies were tested on a panel of 154 established human cancer cell lines and on short term cultures of 10 human fibroblast and kidney epithelial cells (Tables III and IV). The immunopathological specificities of these antibodies were determined on a panel of human adult and fetal tissues, as well as normal colon epithelium and colon cancer tissues from 55 patients (Table V).

Monoclonal antibodies V-215, K-314 and V-715 were obtained after immunization with fresh colon specimen; antibodies A-33, A-37 were obtained after immunization with pancreas cancer cell lines AsPc-1, and antibodies H-15, (HT 29/15 or HT29-15), H-70 and H-26 (HT 29/26 or HT29-26) were obtained after immunization with colon cancer cell line HT-29.

The heavy chain subclass of the eight antibodies are: V-215, gamma-1; A-33, gamma-2b; K-314, gamma-1; A-37, gamma-1; H-15, gamma-1; H-70, mu; H-26, gamma-2a.

## Example II

V-215: Antibody V-215 react with 9/17 colon cancer cell lines with a strongest titer of $5 \times 10^4$ against SW-1417 colon cancer cell line by rosetting.  One lung, one ovarian, one terato-carcinoma and one melanoma cell lines were positive but all 152 other cell ines tested were negative in direct and absorption tests (Table III).  The antigen was detected on secretion of bronchial epithelium and uterine endometrium, but not any other adult or fetal tissues tested (Table IV).  In immunoperoxidase staining of the frozen section from 55 patients, V-215 was negative with normal but positive with colon tumor in 7 patients (Table V).

V-215 antigen was immunoprecipitated from [³H] glucosamine labelled cell extracts from colon cancer cell line SW-1417. The molecular weight is 140000 as estimated by polyacrylamide gel electrophoresis.

## Example III

A-33: Antibody A-33 is an IgG2b antibody that reacts with 5/17 colon carcinomas and 1/3 pancreatic carcinoma (AsPc-1) with a titer of 10. A-33 also reacts with 3/6 T cell leukemia cell lines; all 155 other cell types tested were negative. Correlation between A-33 and T cell related antigens; OKT-6, T37,1, OKT-4, T,11, CL3-3 (13), CL3-40 (13), was examined by the inhibition tests and by rosetting assay on immunizing cell line AsPc-1 and all the antigens were negative in both tests.

Antibody A-33 react with normal adjacent colonic mucosa and carcinoma of the colon cancer patient (Table V). One out of 5 pancreatic mucosa and pancreas cancer of one patient was also positive with the antigen. A-33 did not react with any other tissue sections examined on immunopathology staining (Table IV).

The antigen was not destroyed by heating at 100°C for 5 minutes and it was present in the choloroform/methanol extract of AsPc-1 cells. In immunoprecipitation experiments using cell extracts labelled with [³H]glucosamine, radioactivity was precipitated that migrated at the dye front in 9% acrylamide gels. These properties strongly suggest that the antigen is a lipid.

EXAMPLE IV

K-314: Antibody K-314 reacted with 13/17 colon carcinomas and 3/3 pancreas carcinomas, 7/25 lung carcinomas, 1/10 bladder carcinomas, and 3/5 chorio and teratocarcinomas; the other 137 cell lines tested were negative (Table III).

In tissue sections, antibody K-314 reacted with lung, uterus and heterogeneous population of gastrointestinal tract epithelial cells of normal adult and fetal tissues (Table IV). Among the gastrointestinal cancer patients, K-314 is present in carcinoma but not in normal adjacent mucosa in 11 colon cancer, 5 metastatic colon cancer to the liver and 3 pancreas cancer patients (Table V).

K-314 was immunoprecipitated from [³H]glucosamine labelled cell lysate of AsPc-1. The molecular weight of the antigen is 170000 as estimated by polyacrylamide gel electrophoresis.

## EXAMPLE V

**H-15:** Antibody H-15 (HT-29-15) is an IgGl antibody that reacts with 12/17 colon cancers, 2/3 pancreatic cancers, 2/2 Hepatic and biliary cancers, 4/5 lung cancers, 1/8 bladder cancers, 2/4 ovarian cancers and weak rosetting with one melanoma and one renal cancer cell lines (Table III). H-15 is found in lung and in some proportion of gastrointestinal mucosa in tissue sections (Table IV). H-15 is positive in cancer but negative in normal counterpart of the same patient in 8 colon cancers, 3 metastatic colon cancers and in 2 pancreas cancers (Table V).

The antigen was not destroyed by heating at 100°C for 5 minutes and was proteinase resistant. The antigen disappeared after treatment with neuraminidase. In immunoprecipitation with [³H[ glucosamine, weak broad band of molecular weight over 200000 is observed.

## Example VI

**A-37:** A-37 is present in 5/17 colon cancers and 3/3 pancreatic cancers, 3/20 renal cancers, 3/5 chorio- and teratocarcinomas and in 15/25 hematopoietic cells tumors (Table III). In tissue sections, the antigen was found only on proxymal tubules of the kidney but not in any other tissues tested (Table IV). This antigen is also heat stable, proteinase and neuraminidase resistant.

## Example VII

V-715: V-715 antigen is in 8/17 colon cancers, 5/25 lung cancers, 1/10 bladder cancers and in almost all renal cancer cell lines (Table III). In tissue sections, V-175 is present in proxymal tubules of the kidney, but not in normal gastrointestinal tract cells (Table IV). The antigen is found on 9 colon and pancreas cancer specimen and on 4 normal adjacent colon and pancreas mucosa of those cancer patients (Table V). The antigen is a glycoprotein and the molecular weight is 120000.

The serology pattern with the cell lines and the immunopathology staining pattern with the tissues are very similar to the Adenosine deaminase binding protein which was detected by renal cancer monoclonals (Andy, Robin J., et al. (1984) J. Biol. Chem. 259:12844). Since V-175 is not detected in normal colon, the determinant of V-715 is likely to be the same as the epitope detected by monoclonal S-23.

## Example VIII

H-70: H-70 is in 13/17 colon cancer cell lines and in several other epithelial cancer cell lines. H-70 is also on 3/5 neuroblastoma cell lines. H-70 is detected in epithelial tissues in immunopathology. Immunoprecipitation with [H]glusosamine was performed to determine its molecular weight as 31000.

## Example IX

H-26: Antigen H-26 (HT-29-26,C-26) is in almost all epithelial cancer cell lines but is not present in any neuroblastoma, melanoma or astrocytoma cell lines (Table III).

In immunopathology H-26 is present in all epithelial cells and in kidney, it is present on distal and collecting tubules (Table IV). H-26 is a glycoprotein and its molecular weight is 29000.

Thus normal versus neoplastic cells of the colon, GI, and pancreas can be differentiated and assayed by contacting a speciman from a human patient with each of the monoclonal antibodies of the panel in serial dilution, and observing any antigen antibody reaction by any of the methods cited. Although specific hybridomas producing monoclonal antibody against gastrointestinal cell antigens are presented, it is obvious that the present invention encompasses all the mAbs exhibiting the characteristics described therein, especially the embodiment describing reaction with normal as well as tumor cell antigens of the GI tract.

One of the preferred panels of the invention for colon cancer is:

## SUMMARY OF MOUSE MONOCLONAL ANTIBODIES - COLON PANEL

| Monoclonal Antibody (Ig subclass) | ATCC# | Molecular | Additional notes |
|---|---|---|---|
| AS33 (IgG2b) | HB 8779 | | N Colon/some Colon Ca. |
| AS37 (IgG1) | HB 8778 | | |
| CLH70 (IgG2b) | HB 8245 | Mr29,000 | |
| CLK314 (IgG1) | HB 8780 | | |
| CLT86 (mu) | HB 8252 | | |
| CLT307 (mu) | HB 8251 | | |
| HT29-15 (IgG1) | HB 8246 | Mr 200,000 | |
| HT29-26 (IgG2a) | HB 8247 | Mr40,000 | epithelial cell marker |

Changes in cell antigens are associated with different stages of differentiation and different stages of cancer. Thus this invention technique defined cell antigens associated with differentiation and cancer of the GI tract and the pancreas.

Legend to Table I

Serological reaction of colon panel monoclonal antibodies with human tumor cell lines of various tissues by rosette formation with human red blood cells conjugated with rabbit anti-Ig, Dippold _Supra_

where   0 = no reaction by rosette formation or absorption

2 = positive rosette reaction at less than 1,000 fold dilution antibody supernatant

3 = positive rosette reaction at greater than 1,000 fold dilution antibody supernatant

1 = positive reaction by absorption test only.

If there is no rosette reaction, the absorption test was done.  Thus if a mAb was negative for the rosette reaction but absorbed onto the test antigen system it was deemed to be a positive reaction such that

1 = positive reaction by the absorption test though mAb gives a negative test for rosette formation

i.e. 0 test for rosette reaction is further tested by the absorption test.  Therefore 0 on this table indicates no reaction by either absorption or rosette reactions.  For comparison, mAb 19.9 was obtained from H. Kaprowski and assayed as well alongside the mAbs of the present invention Atkinson, B.F. et al., Cancer Research, 42:4820-4823(1982).

In Table I actual titers are included.

Immunogen for CLT series is Tallevi, for HT and CLH antibodies the immunogen is HT-29.

Legend to Table II

Immunopathological reaction of some of the colon panel monoclonal antibodies with fetal and adult normal human tissue and cancer tissue in frozen section by indirect immunofluorescence.

0 = no reaction

❸ = positive reaction

❷ = heterogeneous reaction within the tissue

The following monoclonal antibody-producing-hybridoma cell lines are maintained on deposit at Sloan-Kettering Institute for Cancer Research, 1275 York Avenue, New York, New York 10021 namely:

V-215, K-314, V-715, As-33, As-37, CLH 70, CLK 314, CLT 86, CLT 307, HT 29-15 and HT 29-26.

## TABLE I

### Serology

Serological Reaction of Monoclonal Antibodies
Produced from Human Colon Tumor Immunogen With Various
Human Cancer Cell Lines

**IMMUNIZING TUMOR: COLON**

| CELLS TESTED | CLH 6 | CLT 85 | CLT 479 | CLT 174 | CLH 68 | CLT 152 | CLH 70 | HT29 -15 | HT29 -26 | HT29 -36 | CLT 218 | CLT 15 | CLT 307 | CLT 86 | 19.9 |
|---|---|---|---|---|---|---|---|---|---|---|---|---|---|---|---|
| **Liver Ca.:** | | | | | | | | | | | | | | | |
| SK-HEP-1 | 1 | 0 | 0 | 0 | 0 | 0 | 2 | 3 | 3 | 0 | 0 | 0 | 0 | 0 | 0 |
| **Biliary duct:** | | | | | | | | | | | | | | | |
| Charles | 0 | 0 | 2 | 0 | 0 | 0 | 0 | 3 | 3 | | 0 | 0 | | 0 | 0 |
| **Astrocytoma:** | | | | | | | | | | | | | | | |
| Goodstein | 0 | 0 | 0 | 0 | 0 | 0 | 0 | 0 | | | 0 | 0 | 0 | 0 | 0 |
| U251 | 0 | 0 | 0 | 0 | 0 | 0 | 0 | 0 | | | 0 | 0 | 0 | | 0 |
| Becker | 0 | 0 | 0 | 0 | 0 | 0 | 0 | 0 | | | 0 | 0 | 0 | | 0 |
| Machino | 0 | 0 | 0 | 0 | 0 | 0 | 0 | 0 | 0 | 0 | 0 | 0 | 0 | 0 | 0 |
| Jones | 0 | 0 | 0 | 0 | 0 | 0 | 0 | 0 | | | 0 | 0 | 0 | | 0 |
| AJ | 0 | 0 | 0 | 0 | 0 | 0 | 0 | 0 | 0 | | 0 | 0 | 0 | | 0 |
| Lear | 0 | 0 | 0 | 0 | 0 | 0 | 0 | 0 | 0 | 0 | 0 | 0 | 0 | 0 | 0 |
| Healy | 0 | 0 | 0 | 0 | 0 | 0 | 0 | 0 | 0 | | 0 | 0 | 0 | 0 | 0 |
| T98 | 0 | 0 | 0 | 0 | 0 | 0 | 0 | 0 | | | 0 | 0 | 0 | 0 | 0 |
| U373 | 0 | 0 | 0 | 0 | 0 | 0 | 0 | 0 | 0 | 0 | 0 | 0 | 0 | 0 | 0 |
| **Melanoma:** | | | | | | | | | | | | | | | |
| SK-MEL-31 (3) | 0 | 0 | 0 | 0 | 0 | 0 | 0 | 2 | 0 | 0 | 0 | 0 | 0 | 0 | 0 |
| -23 | 0 | 0 | 0 | 0 | 0 | 0 | 0 | 0 | 0 | | 0 | 0 | 0 | 0 | 0 |
| -13 | 0 | 0 | 0 | 0 | 0 | 0 | 0 | 0 | 0 | 0 | 0 | 0 | 0 | 0 | 0 |
| -37 | 0 | 0 | 0 | 0 | 0 | 0 | 0 | 2 | 0 | 0 | 0 | 0 | 0 | 0 | 0 |
| -93 (2) | 0 | 0 | 0 | 0 | 3 | 0 | 0 | 3 | 2 | 0 | 0 | 0 | 0 | 0 | 0 |
| -28 | 0 | 0 | 0 | 0 | 0 | 0 | 0 | 0 | 0 | 3 | 0 | 0 | 0 | 0 | 0 |
| MeWo | 0 | 0 | 0 | 0 | 0 | 0 | 0 | 0 | 0 | 0 | 0 | 0 | 0 | 0 | 0 |
| **Neuroblastoma:** | | | | | | | | | | | | | | | |
| SK-N-MC | 0 | 0 | 0 | 0 | 0 | 0 | 3 | 0 | 0 | 0 | 0 | 0 | 0 | 0 | 0 |
| -SH | 0 | 0 | 0 | 0 | 0 | 0 | 3 | 0 | 0 | 0 | 0 | 0 | 0 | 0 | 0 |
| -BE2 | 0 | 0 | 0 | 0 | 0 | 0 | 3 | 0 | 0 | 0 | 0 | 0 | 0 | 0 | 0 |
| LAN-1-5 | 0 | 0 | 0 | 0 | 0 | 0 | 0 | 0 | 0 | 0 | 0 | 0 | 0 | 0 | 0 |
| CHP-234N | | | | | | | | | | | | | | | |

## TABLE I

### Serology

Serological Reaction of Monoclonal Antibodies
Produced from Human Colon Tumor Immunogen With Various
Human Cancer Cell Lines

### IMMUNIZING TUMOR: COLON

| CELLS TESTED | CIH 6 | CLT 85 | CLT 479 | CLT 174 | CIH 68 | CLT 152 | CIH 70 | HT29 -15 | HT29 -26 | HT29 -36 | CLT 218 | CLT 15 | CLT 307 | CLT 86 | 19.9 |
|---|---|---|---|---|---|---|---|---|---|---|---|---|---|---|---|
| **Breast Ca.:** | | | | | | | | | | | | | | | |
| MDA MB 361 | 0 | 0 | 0 | 0 | 0 | 0 | 3 | 3 | 0 | 0 | 0 | 0 | 0 | 0 | 0 |
| MDA MB 231 | 0 | 0 | 0 | 0 | 0 | 0 | 2 | 3 | 3 | 0 | 0 | 0 | 3 | 0 | 0 |
| BT 20 | 0 | 0 | 0 | 0 | 0 | 0 | 0 | 3 | 3 | 0 | 0 | 0 | 0 | 0 | 0 |
| CAMA | 0 | 0 | 1 | 1 | 0 | 0 | 0 | 2 | 0 | | 3 | 3 | 3 | 3 | 0 |
| SK-BR-3 | 0 | 0 | 0 | 0 | 0 | 0 | 0 | 3 | 0 | | 0 | 0 | 0 | 0 | 0 |
| ALAB | 0 | 0 | 0 | 0 | 0 | 0 | 3 | 3 | | | 0 | 0 | 0 | 0 | 0 |
| MCF-7 | 0 | 0 | 0 | 0 | 0 | 0 | 0 | 3 | 3 | 3 | 3 | 0 | 0 | 3 | 0 |
| **Kidney Ca.:** | | | | | | | | | | | | | | | |
| SK-RC-6 | 0 | 0 | 0 | 0 | 0 | 0 | 0 | 0 | 3 | 0 | 0 | 0 | 0 | 0 | |
| -7 | 0 | 0 | 0 | 0 | 0 | 0 | 0 | 0 | 3 | 0 | | | | | 0 |
| -29 | 0 | 0 | 0 | 0 | 0 | 3 | 0 | 0 | 3 | 0 | 0 | 0 | 0 | | 0 |
| -4 | 0 | 0 | 0 | 0 | 0 | 0 | 0 | 0 | 3 | 0 | 0 | 0 | 0 | | |
| **Ovary Ca:** | | | | | | | | | | | | | | | |
| SK-OV-3 | 0 | 0 | 0 | 0 | 0 | 0 | 0 | 0 | 3 | 0 | 0 | 0 | 0 | 0 | 0 |
| ROAC | 0 | 0 | 0 | 0 | 0 | 0 | 3 | 0 | 3 | | 0 | 0 | 0 | 0 | 0 |
| 2774 | 0 | 0 | 0 | 0 | 0 | 0 | 0 | 0 | 3 | | 0 | 0 | 0 | 0 | 0 |
| SW 626 | 0 | 0 | 3 | 3 | 0 | 0 | 0 | 3 | 3 | | 3 | 0 | 3 | 3 | 0 |
| Shustak | 0 | 0 | 0 | 0 | 0 | 3 | 0 | 3 | 3 | | 3 | 3 | 3 | 3 | 0 |
| Turanek | 0 | | 2 | 0 | 0 | 3 | 2 | 3 | 3 | 0 | 0 | 0 | 0 | 3 | 10 |
| **Uterine Ca.:** | | | | | | | | | | | | | | | |
| ME180 | 0 | 0 | 0 | 0 | 0 | 0 | 0 | 0 | 3 | 3 | 0 | 0 | 0 | 0 | 0 |
| **Chorioepithelium:** | | | | | | | | | | | | | | | |
| SVCC | 0 | 0 | 2 | | 0 | 0 | 0 | 0 | 3 | | 0 | 0 | 3 | 0 | 0 |

Table I

## Serology

Serological Reaction of Monoclonal Antibodies
Produced from Human Colon Tumor Immunogen With Various
Human Cancer Cell Lines

### IMMUNIZING TUMOR: COLON

| CELLS TESTED | CLH 6 | CLT 85 | CLT 479 | CLT 174 | CLH 68 | CLT 152 | CLH 70 | HT29 -15 | HT29 -26 | HT29 -36 | CLT 218 | CLT 15 | CLT 307 | CLT 86 | 19.9 |
|---|---|---|---|---|---|---|---|---|---|---|---|---|---|---|---|
| Lung Ca: | | | | | | | | | | | | | | | |
| SK-LC-3 | 0 | 0 | 2 | 0 | 0 | 0 | 3 | 3 | 3 | 0 | 0 | 0 | 0 | 0 | 0 |
| -4 | 0 | 0 | 0 | 0 | 0 | 0 | 0 | 3 | 3 | 2 | 0 | 0 | 0 | 0 | 10 |
| -5 | 1 | 0 | 0 | 0 | 0 | 0 | 0 | 3 | 3 | 0 | 0 | 0 | 0 | 0 | 0 |
| -6 | 0 | 0 | 0 | 0 | 0 | 0 | 3 | 2 | 0 | 0 | 0 | 0 | 2 | 0 | 0 |
| -7 | 0 | 0 | 0 | 0 | 0 | 0 | 0 | 0 | 3 | 2 | 0 | 0 | 0 | 0 | 0 |
| -8 | 0 | 0 | 0 | 2 | 0 | 0 | 0 | 0 | 3 | 3 | 0 | 0 | 0 | 0 | 0 |
| -13 | 0 | 0 | 0 | 0 | 0 | 0 | 2 | 0 | 3 | 2 | 0 | 0 | 0 | 0 | 0 |
| Lawson | 0 | 1 | 1 | 1 | 0 | 3 | 2 | 3 | 3 | 2 | 3 | 0 | 0 | 3 | $10^3$ |
| Bladder Ca: | | | | | | | | | | | | | | | |
| T-24 | 0 | 0 | 0 | 0 | 0 | 0 | 0 | 0 | 0 | 3 | 0 | 0 | 0 | 0 | 0 |
| TCC SUP | 0 | 0 | 0 | 0 | 0 | 0 | 0 | 0 | 0 | 0 | 0 | 0 | 0 | 0 | 0 |
| 253J | 0 | 0 | 0 | 0 | 0 | 0 | 0 | 0 | 0 | 0 | 0 | 3 | 0 | 0 | 0 |
| 639V | 0 | 0 | 0 | 0 | 0 | 0 | 0 | 0 | 3 | 3 | 0 | 0 | 0 | 0 | 0 |
| 486P | 0 | 0 | 0 | 0 | 0 | 0 | 3 | 3 | 3 | 0 | 0 | 0 | 0 | 0 | 0 |

32

Table II

Immunopathology

Normal Tissue Distribution of the Monoclonal
Antibodies Produced from Human
Colon Tumor Immunogen

A. FETAL TISSUES

| | CLT 85 | CLH 6 | HT 29/36 | HT 29/15 | CLT 479 | CLT 15 | CLT 174 | CLT 86 | CLH 70 | CLT 152 | 19.9 |
|---|---|---|---|---|---|---|---|---|---|---|---|
| LUNG | ± | 0 | 0 | 0 | + | + | + | + | + | + | |
| Bronchial Epithelium | ± | 0 | 0 | 0 | ± | ± | ± | + | + | + | |
| Cartilage | 0 | 0 | 0 | 0 | 0 | 0 | 0 | 0 | 0 | 0 | |
| Pneumocytes | 0 | 0 | 0 | 0 | 0 | 0 | 0 | 0 | 0 | 0 | |
| Connect.Tis | 0 | 0 | 0 | 0 | 0 | 0 | 0 | 0 | 0 | 0 | 0 |
| HEART | 0 | 0 | 0 | 0 | 0 | 0 | 0 | 0 | 0 | 0 | 0 |
| THYMUS | 0 | 0 | 0 | 0 | + | 0 | 0 | 0 | 0 | + | + |
| Hassal's C. | 0 | 0 | 0 | 0 | + | 0 | 0 | 0 | 0 | + | + |
| Thymocytes | 0 | 0 | 0 | 0 | 0 | 0 | 0 | 0 | 0 | 0 | 0 |
| SPLEEN | 0 | 0 | 0 | 0 | 0 | 0 | 0 | 0 | 0 | 0 | 0 |
| White Pulp | 0 | 0 | 0 | 0 | 0 | 0 | 0 | 0 | 0 | 0 | 0 |
| Red Pulp | 0 | 0 | 0 | 0 | 0 | 0 | 0 | 0 | 0 | 0 | 0 |
| LIVER | 0 | 0 | 0 | 0 | 0 | 0 | 0 | + | + | + | 0 |
| | 0 | 0 | 0 | 0 | 0 | 0 | 0 | 0 | 0 | 0 | 0 |
| Biliary Epi Cells | 0 | 0 | 0 | 0 | 0 | 0 | 0 | + | + | + | 0 |
| GALLBLAD. | 0 | 0 | 0 | 0 | 0 | 0 | 0 | + | + | + | 0 |
| ESOPHAGUS | 0 | 0 | 0 | 0 | ± | 0 | ± | ± | 0 | ± | ± |
| STOMACH | 0 | ± | 0 | 0 | 0 | 0 | 0 | ± | + | ± | ± |
| SMALL INT. | 0 | 0 | 0 | 0 | 0 | 0 | 0 | 0 | 0 | 0 | 0 |
| COLON | ± | ± | 0 | 0 | ± | ± | ± | + | + | + | ± |
| PANCREAS | 0 | + | 0 | + | + | + | + | + | + | + | + |
| Exocrine | 0 | ± | 0 | + | + | + | + | + | + | + | + |
| Endocrine | 0 | 0 | 0 | 0 | 0 | 0 | 0 | 0 | 0 | 0 | 0 |
| KIDNEY | 0 | + | 0 | 0 | 0 | 0 | 0 | + | + | 0 | 0 |
| Glomerulus | 0 | 0 | 0 | 0 | 0 | 0 | 0 | 0 | 0 | 0 | 0 |
| Prox. Tub. | 0 | 0 | 0 | 0 | 0 | 0 | 0 | 0 | + | 0 | 0 |
| Distal Tub. | 0 | + | 0 | 0 | 0 | 0 | 0 | + | 0 | 0 | 0 |
| Collec. Tub | 0 | + | 0 | 0 | 0 | 0 | 0 | + | 0 | 0 | 0 |
| URETER | 0 | + | 0 | ± | 0 | 0 | 0 | ± | 0 | + | 0 |
| UR. BLAD. | 0 | + | 0 | ± | 0 | 0 | 0 | ± | 0 | + | 0 |
| ADRENAL | 0 | 0 | 0 | 0 | 0 | 0 | 0 | 0 | 0 | 0 | 0 |
| Cortex | 0 | 0 | 0 | 0 | 0 | 0 | 0 | 0 | 0 | 0 | 0 |
| Medulla | 0 | 0 | 0 | 0 | 0 | 0 | 0 | 0 | 0 | 0 | 0 |
| TESTES | 0 | 0 | 0 | 0 | 0 | 0 | 0 | 0 | 0 | 0 | 0 |
| Germ. Cells | 0 | 0 | 0 | 0 | 0 | 0 | 0 | 0 | 0 | 0 | 0 |
| Endoc. Cel. | 0 | 0 | 0 | 0 | 0 | 0 | 0 | 0 | 0 | 0 | 0 |
| Connect. T. | 0 | 0 | 0 | 0 | 0 | 0 | 0 | 0 | 0 | 0 | 0 |

33

TABLE II

SK 340
4/12/85

Immunopathology

Normal Tissue Distribution of the Monoclonal
Antibodies Produced from Human
Colon Tumor Immunogen

A. FETAL TISSUES (Cont'd.)

| | CLT 85 | CLH 28 | CLH 6 | HT 29/36 | HT 29/15 | CLT 479 | CLT 15 | CLT 174 | CLT 86 | CLH 70 | CLT 152 | 19.9 |
|---|---|---|---|---|---|---|---|---|---|---|---|---|
| OVARY | 0 | 0 | 0 | 0 | 0 | 0 | 0 | 0 | 0 | 0 | 0 | 0 |
| Germ. Cells | 0 | 0 | 0 | 0 | 0 | 0 | 0 | 0 | 0 | 0 | 0 | 0 |
| Connect. T. | 0 | 0 | 0 | 0 | 0 | 0 | 0 | 0 | 0 | 0 | 0 | 0 |
| FALLOP. T. | 0 | 0 | 0 | 0 | 0 | 0 | 0 | 0 | 0 | 0 | 0 | 0 |
| UTERUS | 0 | 0 | 0 | 0 | 0 | 0 | 0 | 0 | + | + | 0 | 0 |
| Endometrium | 0 | 0 | 0 | 0 | 0 | 0 | 0 | 0 | + | + | 0 | 0 |
| Myometrium | 0 | 0 | 0 | 0 | 0 | 0 | 0 | 0 | 0 | 0 | 0 | 0 |
| CERVIX | 0 | 0 | 0 | 0 | 0 | 0 | 0 | 0 | + | 0 | 0 | 0 |
| Endocervix | 0 | 0 | 0 | 0 | 0 | 0 | 0 | 0 | + | 0 | 0 | 0 |
| Exocervix | 0 | 0 | 0 | 0 | 0 | 0 | 0 | 0 | ± | 0 | 0 | 0 |
| SKIN | 0 | 0 | 0 | 0 | 0 | 0 | ± | 0 | + | 0 | + | 0 |
| Epidermis | 0 | 0 | 0 | 0 | 0 | 0 | ± | 0 | ± | 0 | ± | 0 |
| Melanocytes | 0 | 0 | 0 | 0 | 0 | 0 | 0 | 0 | 0 | 0 | 0 | 0 |
| Sweat Gland | 0 | 0 | 0 | 0 | 0 | 0 | 0 | 0 | 0 | 0 | 0 | 0 |
| Sebac. Gld. | 0 | 0 | 0 | 0 | 0 | 0 | 0 | 0 | 0 | 0 | 0 | 0 |
| Hair Fol. | 0 | 0 | 0 | 0 | 0 | 0 | 0 | 0 | 0 | 0 | 0 | 0 |
| Dermis C.T. | 0 | 0 | 0 | 0 | 0 | 0 | 0 | 0 | 0 | 0 | 0 | 0 |
| BRAIN | 0 | 0 | 0 | 0 | 0 | 0 | 0 | 0 | 0 | 0 | 0 | 0 |
| Neurons | 0 | 0 | 0 | 0 | 0 | 0 | 0 | 0 | 0 | 0 | 0 | 0 |
| Glial Cells | 0 | 0 | 0 | 0 | 0 | 0 | 0 | 0 | 0 | 0 | 0 | 0 |
| Dendrites | 0 | 0 | 0 | 0 | 0 | 0 | 0 | 0 | 0 | 0 | 0 | 0 |
| LYMPH NODE | 0 | 0 | 0 | 0 | 0 | 0 | 0 | 0 | 0 | 0 | 0 | 0 |
| BLOOD VES. | 0 | 0 | 0 | 0 | 0 | 0 | 0 | 0 | 0 | 0 | 0 | 0 |
| Endoth. Cel. | 0 | 0 | 0 | 0 | 0 | 0 | 0 | 0 | 0 | 0 | 0 | 0 |
| Smooth Ms. | 0 | 0 | 0 | 0 | 0 | 0 | 0 | 0 | 0 | 0 | 0 | 0 |
| SOFT TIS. | 0 | 0 | 0 | 0 | 0 | 0 | 0 | 0 | 0 | 0 | 0 | 0 |
| SECRETION | ± | 0 | 0 | 0 | 0 | + | 0 | + | + | + | + | + |

## TABLE II

### Immunopathology

Normal Tissue Distribution of the Monoclonal
Antibodies Produced from Human
Colon Tumor Immunogen

### A. FETAL TISSUES (CONT'D.)

| | CLT 307 | CLT 218 | HT 29/26 | CLH 68 |
|---|---|---|---|---|
| LUNG | + | + | + | 0 |
| Bronchial Epithelium | + | + | + | 0 |
| Cartilage | 0 | 0 | 0 | 0 |
| Pneumocytes | 0 | 0 | 0 | 0 |
| Connect. Tis | 0 | 0 | 0 | 0 |
| HEART | 0 | 0 | 0 | 0 |
| THYMUS | 0 | 0 | 0 | + |
| Hassal's C. | 0 | 0 | 0 | + |
| Thymocytes | 0 | 0 | 0 | 0 |
| SPLEEN | 0 | 0 | 0 | 0 |
| White Pulp | 0 | 0 | 0 | 0 |
| Red Pulp | 0 | 0 | 0 | 0 |
| LIVER | + | + | + | + |
| Hepatocytes | 0 | 0 | 0 | 0 |
| Biliary Epi Cells | + | + | + | + |
| GALLBLAD. | + | + | + | + |
| ESOPHAGUS | + | ± | ± | ± |
| STOMACH | ± | + | 0 | + |
| SMALL INT. | 0 | 0 | ± | + |
| COLON | + | + | + | + |
| PANCREAS | + | + | + | 0 |
| Exocrine | + | + | + | 0 |
| Endocrine | 0 | 0 | 0 | 0 |
| KIDNEY | 0 | + | + | 0 |
| Glomerulus | 0 | 0 | 0 | 0 |
| Prox. Tub. | 0 | 0 | 0 | 0 |
| Distal Tub. | 0 | + | + | 0 |
| Collec. Tub | 0 | + | + | 0 |
| URETER | + | + | + | + |
| UR. BLAD. | + | + | + | + |
| ADRENAL | 0 | 0 | 0 | 0 |
| Cortex | 0 | 0 | 0 | 0 |
| Medulla | 0 | 0 | 0 | 0 |
| TESTES | 0 | 0 | 0 | 0 |
| Germ. Cells | 0 | 0 | 0 | 0 |
| Endoc. Cel. | 0 | 0 | 0 | 0 |
| Connect. T. | 0 | 0 | 0 | 0 |

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

MONOCLONAL ANTIBODIES

| NORMAL FETAL TISSUES | 85 | 28 | 6 | 29/36 | 29/15 | 479 | 15 | 174 | 86 | 70 | 152 | | 307 | 218 | 29/26 | 68 |
|---|---|---|---|---|---|---|---|---|---|---|---|---|---|---|---|---|
| KIDNEY | 0 | 0 | ● | 0 | 0 | 0 | 0 | 0 | ● | ● | 0 | 0 | 0 | ● | ● | ● |
|   Glomerulus | 0 | 0 | 0 | 0 | 0 | 0 | 0 | 0 | 0 | 0 | 0 | 0 | 0 | 0 | 0 | 0 |
|   Proximal Tubules | 0 | 0 | 0 | 0 | 0 | 0 | 0 | 0 | 0 | ● | 0 | 0 | 0 | ● 0 | 0 | 0 |
|   Distal Tubules | 0 | 0 | ● | 0 | 0 | 0 | 0 | 0 | ● | 0 | 0 | 0 | 0 | ● | ● | 0 |
|   Collecting Tubules | 0 | 0 | ● | 0 | 0 | 0 | 0 | 0 | ● | 0 | 0 | 0 | 0 | ● | ● | 0 |
| URETER | 0 | 0 | ● | 0 | ● | 0 | 0 | 0 | ● | 0 | ● | 0 | ● | ● | ● | ● |
| URINARY BLADDER | 0 | 0 | ● | 0 | ● | 0 | 0 | 0 | ● | 0 | ● | 0 | ● | ● | ● | ● |
| ADRENAL | 0 | 0 | 0 | 0 | 0 | 0 | 0 | 0 | 0 | 0 | 0 | 0 | 0 | 0 | 0 | 0 |
|   Cortex | 0 | 0 | 0 | 0 | 0 | 0 | 0 | 0 | 0 | 0 | 0 | 0 | 0 | 0 | 0 | 0 |
|   Medulla | 0 | 0 | 0 | 0 | 0 | 0 | 0 | 0 | 0 | 0 | 0 | 0 | 0 | 0 | 0 | 0 |
| TESTES | 0 | 0 | 0 | 0 | 0 | 0 | 0 | 0 | 0 | 0 | 0 | 0 | 0 | 0 | 0 | 0 |
|   Germ Cells | 0 | 0 | 0 | 0 | 0 | 0 | 0 | 0 | 0 | 0 | 0 | 0 | 0 | 0 | 0 | 0 |
|   Endocrine Cells | 0 | 0 | 0 | 0 | 0 | 0 | 0 | 0 | 0 | 0 | 0 | 0 | 0 | 0 | 0 | 0 |
|   Connective Tissue | 0 | 0 | 0 | 0 | 0 | 0 | 0 | 0 | 0 | 0 | 0 | 0 | 0 | 0 | 0 | 0 |
| OVARY | 0 | 0 | 0 | 0 | 0 | 0 | 0 | 0 | 0 | 0 | 0 | 0 | 0 | 0 | 0 | 0 |
|   Germ Cells | 0 | 0 | 0 | 0 | 0 | 0 | 0 | 0 | 0 | 0 | 0 | 0 | 0 | 0 | 0 | 0 |
|   Connective Tissue | 0 | 0 | 0 | 0 | 0 | 0 | 0 | 0 | 0 | 0 | 0 | 0 | 0 | 0 | 0 | 0 |
| FALLOPIAN TUBES | 0 | 0 | 0 | 0 | 0 | 0 | 0 | 0 | 0 | 0 | 0 | 0 | 0 | 0 | ● | 0 |
| UTERUS | 0 | 0 | 0 | 0 | 0 | 0 | 0 | 0 | ● | ● | 0 | 0 | 0 | ● | ● | 0 |
|   Endometrium | 0 | 0 | 0 | 0 | 0 | 0 | 0 | 0 | ● | ● | 0 | 0 | 0 | ● | ● | 0 |
|   Myometrium | 0 | 0 | 0 | 0 | 0 | 0 | 0 | 0 | 0 | 0 | 0 | 0 | 0 | 0 | 0 | 0 |
| CERVIX | 0 | 0 | 0 | 0 | 0 | 0 | 0 | 0 | ● | 0 | 0 | 0 | 0 | 0 | ● | ● |
|   Endocervix | 0 | 0 | 0 | 0 | 0 | 0 | 0 | 0 | ● | 0 | 0 | 0 | 0 | 0 | ● | 0 |
|   Exocervix | 0 | 0 | 0 | 0 | 0 | 0 | 0 | 0 | ● | 0 | 0 | 0 | 0 | 0 | 0 | ● |

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

### MONOCLONAL ANTIBODIES

| NORMAL FETAL TISSUES | 85 | 23 | 5 | 29/36 | 29/15 | 479 | 15 | 174 | 86 | 70 | 152 | | 307 | 218 | 29/26 | 68 |
|---|---|---|---|---|---|---|---|---|---|---|---|---|---|---|---|---|
| LUNG | ● | 0 | 0 | 0 | 0 | ● | ● | ● | ● | ● | ● | 0 | ● | ● | ● | 0 |
|   Bronchial Epithelium | ● | 0 | 0 | 0 | 0 | ● | ● | ● | ● | ● | ● | 0 | ● | ● | ● | 0 |
|   Cartilage | 0 | 0 | 0 | 0 | 0 | 0 | 0 | 0 | 0 | 0 | 0 | 0 | 0 | 0 | 0 | 0 |
|   Pneumocytes | 0 | 0 | 0 | 0 | 0 | 0 | 0 | 0 | 0 | 0 | 0 | 0 | 0 | 0 | 0 | 0 |
|   Connective Tissue | 0 | 0 | 0 | 0 | 0 | 0 | 0 | 0 | 0 | 0 | 0 | 0 | 0 | 0 | 0 | 0 |
| HEART | 0 | 0 | 0 | 0 | 0 | 0 | 0 | 0 | 0 | 0 | 0 | 0 | 0 | 0 | 0 | 0 |
| THYMUS | 0 | 0 | 0 | 0 | 0 | ● | 0 | 0 | 0 | 0 | ● | ● | 0 | 0 | 0 | ● |
|   Hassall's Corpuscles | 0 | 0 | 0 | 0 | 0 | ● | 0 | 0 | 0 | 0 | ● | ● | 0 | 0 | 0 | ● |
|   Thymocytes | 0 | 0 | 0 | 0 | 0 | 0 | 0 | 0 | 0 | 0 | 0 | 0 | 0 | 0 | 0 | 0 |
| SPLEEN | 0 | 0 | 0 | 0 | 0 | 0 | 0 | 0 | 0 | 0 | 0 | 0 | 0 | 0 | 0 | 0 |
|   White pulp | 0 | 0 | 0 | 0 | 0 | 0 | 0 | 0 | 0 | 0 | 0 | 0 | 0 | 0 | 0 | 0 |
|   Red pulp | 0 | 0 | 0 | 0 | 0 | 0 | 0 | 0 | 0 | 0 | 0 | 0 | 0 | 0 | 0 | 0 |
| LIVER | 0 | 0 | 0 | 0 | 0 | 0 | 0 | 0 | ● | ● | ● | 0 | ● | ● | ● | ● |
|   Hepatocytes | 0 | 0 | 0 | 0 | 0 | 0 | 0 | 0 | 0 | 0 | 0 | 0 | 0 | 0 | 0 | 0 |
|   Biliary Epith. Cells | 0 | 0 | 0 | 0 | 0 | 0 | 0 | 0 | ● | ● | ● | 0 | ● | ● | ● | ● |
| GALLBLADDER | 0 | 0 | 0 | 0 | 0 | 0 | 0 | 0 | ● | ● | ● | 0 | ● | ● | ● | ● |
| ESOPHAGUS | 0 | 0 | 0 | 0 | 0 | ● | 0 | ● | ● | 0 | ● | ● | ● | ● | ● | ● |
| STOMACH | 0 | 0 | ● | 0 | 0 | 0 | 0 | 0 | ● | ● | ● | ● | ● | ● | ● | ● |
| SMALL INTESTINE | 0 | 0 | 0 | 0 | 0 | 0 | 0 | 0 | 0 | 0 | ● | 0 | 0 | 0 | ● | ● |
| COLON | ● | 0 | ● | 0 | 0 | ● | ● | ● | ● | ● | ● | ● | ● | ● | ● | ● |
| PANCREAS | 0 | 0 | ● | 0 | ● | ● | ● | ● | ● | ● | ● | ● | ● | ● | ● | 0 |
|   Exocrine | 0 | 0 | ● | 0 | ● | ● | ● | ● | ● | ● | ● | 0 | ● | ● | ● | 0 |
|   Endocrine | 0 | 0 | 0 | 0 | 0 | 0 | 0 | 0 | 0 | 0 | 0 | 0 | 0 | 0 | 0 | 0 |

## TABLE IIA

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

MONOCLONAL ANTIBODIES

| NORMAL FETAL TISSUES | 85 | 28 | 6 | 29/36 | 29/15 | 479 | 15 | 174 | 86 | 70 | 152 | | 307 | 218 | 29/26 | 68 | |
|---|---|---|---|---|---|---|---|---|---|---|---|---|---|---|---|---|---|
| SKIN | 0 | 0 | 0 | 0 | 0 | 0 | 8 | 0 | 8 | 0 | 8 | 0 | 8 | 8 | 8 | 8 | 8 |
| Epidermis | 0 | 0 | 0 | 0 | 0 | 0 | 8 | 0 | 8 | 0 | 8 | 0 | 8 | 8 | 8 | 9 | |
| Melanocytes | 0 | 0 | 0 | 0 | 0 | 0 | 0 | 0 | 0 | 0 | 0 | 0 | 0 | 0 | 0 | U | |
| Sweat Gland | 0 | 0 | 0 | 0 | 0 | 0 | 0 | 0 | 0 | 0 | 0 | 0 | 0 | 0 | 8 | U | |
| Sebaceous Gland | 0 | 0 | 0 | 0 | 0 | 0 | 0 | 0 | 0 | 0 | 0 | 0 | 0 | 0 | 0 | 0 | |
| Hair Follicle | 0 | 0 | 0 | 0 | 0 | 0 | 0 | 0 | 0 | 0 | 0 | 0 | 0 | 0 | 0 | 0 | |
| Dermis Connective Tissue | 0 | 0 | 0 | 0 | 0 | 0 | 0 | 0 | 0 | 0 | 0 | 0 | 0 | 0 | 0 | 0 | |
| | | | | | | | | | | | | | | | | | |
| BRAIN | 0 | 0 | 0 | 0 | 0 | 0 | 0 | 0 | 0 | 0 | 0 | 0 | 0 | 0 | 0 | 0 | |
| Neurons | 0 | 0 | 0 | 0 | 0 | 0 | 0 | 0 | U | 0 | 0 | 0 | 0 | 0 | U | 0 | |
| Glial Cells | 0 | 0 | 0 | 0 | 0 | 0 | 0 | 0 | 0 | 0 | 0 | 0 | 0 | 0 | 0 | 0 | |
| Dendrites | 0 | 0 | 0 | 0 | 0 | 0 | 0 | 0 | 0 | 0 | 0 | 0 | 0 | 0 | 0 | 0 | |
| | | | | | | | | | | | | | | | | | |
| LYMPH NODE | 0 | 0 | 0 | 0 | 0 | 0 | 0 | 0 | 0 | 0 | 0 | 0 | 0 | 0 | 0 | 0 | |
| | | | | | | | | | | | | | | | | | |
| BLOOD VESSEL | 0 | 0 | 0 | 0 | 0 | 0 | 0 | 0 | 0 | 0 | 0 | 0 | 0 | 0 | 0 | 0 | |
| Endothelial Cells | 0 | 0 | 0 | 0 | 0 | 0 | 0 | 0 | 0 | 0 | 0 | 0 | 0 | 0 | 0 | 0 | |
| Smooth Muscle | 0 | 0 | 0 | 0 | 0 | 0 | 0 | 0 | 0 | 0 | 0 | 0 | 0 | 0 | 0 | 0 | |
| | | | | | | | | | | | | | | | | | |
| SOFT TISSUE | 0 | 0 | 0 | 0 | 0 | 0 | 0 | 0 | 0 | 0 | 0 | 0 | 0 | 0 | 0 | 0 | |
| | | | | | | | | | | | | | | | | | |
| SECRETION | 8 | 0 | 0 | 0 | 0 | 8 | 0 | 8 | 8 | 8 | 8 | 8 | 8 | 8 | 8 | 8 | |

SK 340
4/12/85

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

MONOCLONAL ANTIBODIES

| NORMAL ADULT TISSUE | 85 | 28 | 6 | 29/36 | 29/15 | 479 | 15 | 174 | 86 | 70 | 152 | 19.9 | 307 | 218 | 29/26 | 68 |
|---|---|---|---|---|---|---|---|---|---|---|---|---|---|---|---|---|
| KIDNEY | 0 | 0 | 0 | 0 | 0 | 0 | 0 | 0 | 0 | 0 | ● | 0 | 0 | 0 | ● | 0 |
| Glomerulus | 0 | 0 | 0 | 0 | 0 | 0 | 0 | 0 | 0 | 0 | 0 | 0 | 0 | 0 | 0 | 0 |
| Proximal Tubules | 0 | 0 | 0 | 0 | 0 | 0 | 0 | 0 | 0 | 0 | 0 | 0 | 0 | 0 | ● | 0 |
| Henle's Loop | 0 | 0 | 0 | 0 | 0 | 0 | 0 | 0 | 0 | 0 | ● | 0 | 0 | 0 | ● | 0 |
| Distal Tubules | 0 | 0 | 0 | 0 | 0 | 0 | 0 | 0 | 0 | 0 | ● | 0 | 0 | 0 | ● | 0 |
| Collecting Tubules | 0 | 0 | 0 | 0 | 0 | 0 | 0 | 0 | 0 | 0 | ● | 0 | 0 | ● | ● | ● |
| URETER | 0 | 0 | 0 | 0 | 0 | 0 | 0 | ● | 0 | 0 | 0 | 0 | ● | ● | ● | ● |
| URINARY BLADDER | 0 | 0 | 0 | 0 | 0 | 0 | 0 | ● | 0 | 0 | 0 | 0 | ● | ● | ● | ● |
| ADRENAL | 0 | 0 | 0 | 0 | 0 | 0 | 0 | 0 | 0 | 0 | 0 | 0 | 0 | 0 | 0 | 0 |
| Cortex | 0 | 0 | 0 | 0 | 0 | 0 | 0 | 0 | 0 | 0 | 0 | 0 | 0 | 0 | 0 | 0 |
| Medulla | 0 | 0 | 0 | 0 | 0 | 0 | 0 | 0 | 0 | 0 | 0 | 0 | 0 | 0 | 0 | 0 |
| THYROID | 0 | 0 | 0 | 0 | 0 | 0 | 0 | 0 | 0 | 0 | 0 | 0 | 0 | 0 | 0 | 0 |
| Epithelium | 0 | 0 | 0 | 0 | 0 | 0 | 0 | 0 | 0 | 0 | 0 | 0 | 0 | 0 | 0 | 0 |
| Colloid | 0 | 0 | 0 | 0 | 0 | 0 | 0 | 0 | 0 | 0 | 0 | 0 | 0 | 0 | 0 | 0 |
| BREAST | 0 | 0 | 0 | 0 | ● | 0 | 0 | ● | 0 | 0 | ● | ● | ● | ● | ● | ● |
| Duct Cells | 0 | 0 | 0 | 0 | ● | 0 | 0 | ● | 0 | 0 | ● | ● | ● | ● | ● | 0 |
| Acinar Cells | 0 | 0 | 0 | 0 | ● | 0 | 0 | 0 | 0 | 0 | 0 | 0 | 0 | 0 | 0 | 0 |
| Connective Tissue | 0 | 0 | 0 | 0 | 0 | 0 | 0 | 0 | 0 | 0 | 0 | 0 | 0 | 0 | 0 | ● |
| PROSTATE | 0 | 0 | 0 | 0 | 0 | 0 | 0 | 0 | 0 | ● | 0 | ● | 0 | ● | ● | ● |
| Epithelium | 0 | 0 | 0 | 0 | 0 | 0 | 0 | 0 | 0 | ● | 0 | ● | 0 | 0 | ● | 0 |
| Stroma | 0 | 0 | 0 | 0 | 0 | 0 | 0 | 0 | 0 | 0 | 0 | 0 | 0 | 0 | 0 | 0 |
| TESTES | 0 | 0 | 0 | 0 | 0 | 0 | 0 | 0 | 0 | 0 | 0 | 0 | 0 | 0 | 0 | 0 |
| Germ Cells | 0 | 0 | 0 | 0 | 0 | 0 | 0 | 0 | 0 | 0 | 0 | 0 | 0 | 0 | 0 | 0 |
| Endocrine Cells | 0 | 0 | 0 | 0 | 0 | 0 | 0 | 0 | 0 | 0 | 0 | 0 | 0 | 0 | 0 | 0 |
| Connective Tissue | 0 | 0 | 0 | 0 | 0 | 0 | 0 | 0 | 0 | 0 | 0 | 0 | 0 | 0 | 0 | 0 |

Table II A

SK 340
4/12/85

## SUMMARY OF TISSUE DISTRIBUTION AND REACTIVITY OF MONOCLONAL ANTIBODIES AGAINST
## COLON CANCER BY IMMUNOPATHOLOGY IN NORMAL FETAL, NORMAL ADULT AND CANCER TISSUE IN HUMAN SPECIMENS

### MONOCLONAL ANTIBODIES

| NORMAL ADULT TISSUES | 85 | 28 | 6 | 29/36 | 29/15 | 479 | 15 | 174 | 86 | 70 | 152 | 19.9 | 307 | 218 | 29/26 | 68 |
|---|---|---|---|---|---|---|---|---|---|---|---|---|---|---|---|---|
| LUNG | 0 | 0 | 0 | 0 | 0 | 0 | 0 | 0 | ● | 0 | 0 | ● | 0 | ● | ● | 0 |
| Bronchial Epithelium | 0 | 0 | 0 | 0 | 0 | 0 | 0 | 0 | ● | 0 | 0 | ● | 0 | ● | ● | 0 |
| Cartilage | 0 | 0 | 0 | 0 | 0 | 0 | 0 | 0 | 0 | 0 | 0 | 0 | 0 | 0 | 0 | 0 |
| Glandular Epithelium | 0 | 0 | 0 | 0 | 0 | 0 | 0 | 0 | ● | 0 | 0 | ● | 0 | ● | ● | 0 |
| Pneumocytes | 0 | 0 | 0 | 0 | 0 | 0 | 0 | 0 | 0 | 0 | 0 | 0 | 0 | 0 | 0 | 0 |
| Connective Tissue | 0 | 0 | 0 | 0 | 0 | 0 | 0 | 0 | 0 | 0 | 0 | 0 | 0 | 0 | 0 | 0 |
| HEART MUSCLE | 0 | 0 | 0 | 0 | 0 | 0 | 0 | 0 | 0 | 0 | 0 | 0 | 0 | 0 | 0 | 0 |
| SPLEEN | 0 | 0 | 0 | 0 | 0 | 0 | 0 | 0 | 0 | 0 | 0 | 0 | 0 | 0 | 0 | ● |
| White pulp | 0 | 0 | 0 | 0 | 0 | 0 | 0 | 0 | 0 | 0 | 0 | 0 | 0 | 0 | 0 | ● |
| Red pulp | 0 | 0 | 0 | 0 | 0 | 0 | 0 | 0 | 0 | 0 | 0 | 0 | 0 | 0 | 0 | 0 |
| LIVER | 0 | 0 | 0 | 0 | 0 | 0 | 0 | ● | 0 | ● | ● | ● | ● | ● | ● | 0 |
| Hepatocytes | 0 | 0 | 0 | 0 | 0 | 0 | 0 | 0 | 0 | 0 | 0 | 0 | 0 | 0 | 0 | 0 |
| Biliary Epithelium | 0 | 0 | 0 | 0 | 0 | 0 | 0 | ● | 0 | ● | ● | ● | ● | ● | ● | 0 |
| Sinusoids | 0 | 0 | 0 | 0 | 0 | 0 | 0 | 0 | 0 | 0 | 0 | 0 | 0 | 0 | 0 | 0 |
| GALLBLADDER | 0 | 0 | 0 | 0 | 0 | 0 | 0 | ● | 0 | ● | ● | ● | ● | ● | ● | ● |
| ESOPHAGUS | 0 | 0 | 0 | 0 | 0 | 0 | ● | ● | 0 | 0 | ● | ● | ● | ● | 0 | ● |
| STOMACH | 0 | 0 | 0 | 0 | 0 | 0 | 0 | 0 | ● | ● | ● | ● | ● | 0 | 0 | ● |
| SMALL INTESTINE | 0 | 0 | 0 | 0 | 0 | 0 | ● | 0 | ● | ● | ● | 0 | ● | ● | 0 | ● |
| COLON | 0 | 0 | 0 | 0 | 0 | 0 | ● | 0 | ● | ● | ● | 0 | ● | ● | ● | ● |
| G.I. smooth muscle | 0 | 0 | 0 | 0 | 0 | 0 | 0 | 0 | 0 | 0 | 0 | 0 | 0 | 0 | 0 | ● |
| PANCREAS | 0 | 0 | 0 | 0 | ● | ● | ● | ● | ● | ● | ● | ● | ● | ● | ● | 0 |
| Exocrine | 0 | 0 | 0 | 0 | ● | ● | ● | ● | ● | ● | ● | ● | ● | ● | ● | 0 |
| Endocrine | 0 | 0 | 0 | 0 | 0 | 0 | 0 | 0 | 0 | 0 | 0 | 0 | 0 | 0 | 0 | 0 |

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

MONOCLONAL ANTIBODIES

| NORMAL ADULT TISSUES | 85 | 28 | 6 | 29/36 | 29/15 | 479 | 15 | 174 | 86 | 70 | 152 | 19.9 | 307 | 218 | 29/26 | 68 |
|---|---|---|---|---|---|---|---|---|---|---|---|---|---|---|---|---|
| OVARY | 0 | 0 | 0 | 0 | 0 | 0 | 0 | 0 | 0 | 0 | 0 | 0 | 0 | 0 | 0 | 0 |
| Germ Cells | 0 | 0 | 0 | 0 | 0 | 0 | 0 | 0 | 0 | 0 | 0 | 0 | 0 | 0 | 0 | 0 |
| Connective Tissue | 0 | 0 | 0 | 0 | 0 | 0 | 0 | 0 | 0 | 0 | 0 | 0 | 0 | 0 | 0 | 0 |
| FALLOPIAN TUBES | 0 | 0 | 0 | 0 | 0 | 0 | 0 | 0 | 0 | 0 | 0 | 0 | 0 | 0 | 0 | 0 |
| UTERUS | 0 | 0 | 0 | 0 | 0 | 0 | 0 | 0 | 0 | 0 | 0 | 0 | 0 | 0 | ● | 0 |
| Endometrium | 0 | 0 | 0 | 0 | 0 | 0 | 0 | 0 | 0 | 0 | 0 | 0 | 0 | 0 | ● | 0 |
| Myometrium | 0 | 0 | 0 | 0 | 0 | 0 | 0 | 0 | 0 | 0 | 0 | 0 | 0 | 0 | 0 | 0 |
| CERVIX | 0 | 0 | 0 | 0 | 0 | 0 | 0 | 0 | 0 | ● | 0 | ● | 0 | 0 | ● | ● |
| Endocervix | 0 | 0 | 0 | 0 | 0 | 0 | 0 | 0 | 0 | ● | 0 | ● | 0 | 0 | ● | 0 |
| Exocervix | 0 | 0 | 0 | 0 | 0 | 0 | 0 | 0 | 0 | 0 | 0 | 0 | 0 | 0 | ● | ● |
| PLACENTA | 0 | 0 | 0 | 0 | 0 | 0 | 0 | 0 | 0 | 0 | 0 | 0 | 0 | 0 | 0 | 0 |
| Cytotrophoblasts | 0 | 0 | 0 | 0 | 0 | 0 | 0 | 0 | 0 | 0 | 0 | 0 | 0 | 0 | 0 | 0 |
| Syncytotrophoblasts | 0 | 0 | 0 | 0 | 0 | 0 | 0 | 0 | 0 | 0 | 0 | 0 | 0 | 0 | 0 | 0 |
| Sinusoids | 0 | 0 | 0 | 0 | 0 | 0 | 0 | 0 | 0 | 0 | 0 | 0 | 0 | 0 | 0 | 0 |
| SKIN | 0 | 0 | 0 | 0 | ● | ● | 0 | ● | ● | ● | ● | 0 | 0 | ● | ● | 0 |
| Epidermis | 0 | 0 | 0 | 0 | 0 | 0 | 0 | 0 | 0 | 0 | 0 | 0 | 0 | 0 | ● | 0 |
| Melanocytes | 0 | 0 | 0 | 0 | 0 | 0 | 0 | 0 | 0 | 0 | 0 | 0 | 0 | 0 | 0 | 0 |
| Sweat Gland | 0 | 0 | 0 | 0 | ● | ● | 0 | ● | ● | ● | ● | 0 | 0 | ● | ● | 0 |
| Sebaceous Gland | 0 | 0 | 0 | 0 | ● | 0 | 0 | 0 | 0 | ● | 0 | 0 | 0 | ● | 0 | 0 |
| Dermis Connective Tis. | 0 | 0 | 0 | 0 | 0 | 0 | 0 | 0 | 0 | 0 | 0 | 0 | 0 | 0 | 0 | 0 |
| BRAIN | 0 | 0 | 0 | 0 | 0 | 0 | 0 | 0 | 0 | 0 | 0 | 0 | 0 | 0 | 0 | 0 |
| Neurons | 0 | 0 | 0 | 0 | 0 | 0 | 0 | 0 | 0 | 0 | 0 | 0 | 0 | 0 | 0 | 0 |
| Glial Cells | 0 | 0 | 0 | 0 | 0 | 0 | 0 | 0 | 0 | 0 | 0 | 0 | 0 | 0 | 0 | 0 |
| Dendrites | 0 | 0 | 0 | 0 | 0 | 0 | 0 | 0 | 0 | 0 | 0 | 0 | 0 | 0 | 0 | 0 |
| LYMPH NODE | 0 | 0 | 0 | 0 | 0 | 0 | 0 | 0 | 0 | 0 | 0 | 0 | 0 | 0 | 0 | 0 |

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

MONOCLONAL ANTIBODIES

| NORMAL ADULT TISSUES | 85 | 28 | 6 | 29/36 | 29/15 | 479 | 15 | 174 | 86 | 70 | 152 | 19.9 | 307 | 218 | 29/26 | 68 |
|---|---|---|---|---|---|---|---|---|---|---|---|---|---|---|---|---|
| BLOOD VESSEL | 0 | 0 | 0 | 0 | 0 | 0 | 0 | 0 | 0 | 0 | 0 | 0 | 0 | 0 | 0 | 0 |
| Endothelium | 0 | 0 | 0 | 0 | 0 | 0 | 0 | 0 | 0 | 0 | 0 | 0 | 0 | 0 | 0 | 0 |
| Smooth Muscle | 0 | 0 | 0 | 0 | 0 | 0 | 0 | 0 | 0 | 0 | 0 | 0 | 0 | 0 | 0 | 0 |
| CAPILLARIES | 0 | 0 | 0 | 0 | 0 | 0 | 0 | 0 | 0 | 0 | 0 | 0 | 0 | 0 | 0 | 0 |
| SKELETAL MUSCLE | 0 | 0 | 0 | 0 | 0 | 0 | 0 | 0 | 0 | 0 | 0 | 0 | 0 | 0 | 0 | 0 |
| SOFT TISSUE | 0 | 0 | 0 | 0 | 0 | 0 | 0 | 0 | 0 | 0 | 0 | 0 | 0 | 0 | 0 | 0 |
| SECRETIONS | 0 | 0 | ● | ● | ● | ● | ● | ● | ● | ● | ● | ● | ● | ● | ● | ● |

41

SUMMARY OF TISSUE DISTRIBUTION AND REACTIVITY OF MONOCLONAL ANTIBODIES AGAINST
COLON CANCER BY IMMUNOPATHOLOGY IN NORMAL FETAL, NORMAL ADULT AND CANCER TISSUE IN HUMAN SPECIMENS

MONOCLONAL ANTIBODIES

| CANCER TISSUE | 85 | 26 | 6 | 29/36 | 29/15 | 479 | 15 | 174 | 86 | 70 | 152 | 307 | 218 | 29/26 | 68 |
|---|---|---|---|---|---|---|---|---|---|---|---|---|---|---|---|
| COLON CANCER | ● | | 0 | | ● | ● | ● | ● | ● | | ● | | | | ● |
| | 0 | | ● | | 0 | 0 | 0 | 0 | ● | | ● | | | | ● |
| | ● | | ● | | 0 | ● | ● | ● | ● | | ● | | | | ● |
| | ● | | ● | | ● | ● | ● | ● | ● | | ● | | | | ● |
| | 0 | | ● | | 0 | 0 | 0 | 0 | ● | | 0 | | | | ● |
| LUNG CANCER | 0 | | 0 | | 0 | 0 | 0 | 0 | ● | | 0 | | | | ● |
| | 0 | | 0 | | 0 | 0 | 0 | 0 | ● | | 0 | | | | ● |
| | 0 | | 0 | | 0 | 0 | 0 | 0 | ● | | 0 | | | | ● |
| | 0 | | 0 | | 0 | 0 | ● | 0 | ● | | ● | | | | ● |
| | 0 | | 0 | | ● | ● | ● | ● | ● | | ● | | | | ● |
| BREAST CANCER | 0 | | 0 | | 0 | 0 | 0 | 0 | 0 | | 0 | | | | ● |
| | 0 | | ● | | 0 | 0 | 0 | 0 | 0 | | 0 | | | | ● |
| | 0 | | 0 | | 0 | 0 | 0 | 0 | ● | | 0 | | | | ● |
| | 0 | | 0 | | 0 | 0 | 0 | 0 | ● | | 0 | | | | ● |
| | 0 | | 0 | | 0 | 0 | 0 | 0 | 0 | | 0 | | | | ● |
| BLADDER CANCER | 0 | | 0 | | 0 | 0 | 0 | 0 | ● | | ● | | | | ● |
| | 0 | | 0 | | 0 | 0 | 0 | 0 | ● | | 0 | | | | 0 |
| | 0 | | 0 | | 0 | 0 | 0 | 0 | ● | | ● | | | | ● |
| | 0 | | 0 | | 0 | 0 | 0 | 0 | 0 | | 0 | | | | ● |
| | 0 | | 0 | | 0 | 0 | 0 | 0 | ● | | ● | | | | ● |
| | 0 | | 0 | | 0 | 0 | 0 | 0 | ● | | ● | | | | ● |
| TERATOCARCINOMA | 0 | | 0 | | ● | ● | ● | ● | ● | | ● | | | | ● |
| | 0 | | 0 | | ● | ● | ● | ● | ● | | ● | | | | ● |
| | 0 | | 0 | | 0 | 0 | 0 | 0 | ● | | 0 | | | | ● |
| MELANOMA | 0 | | 0 | | 0 | 0 | 0 | 0 | 0 | | 0 | | | | 0 |
| | 0 | | 0 | | 0 | 0 | 0 | 0 | 0 | | 0 | | | | 0 |

# SUMMARY OF TISSUE DISTRIBUTION AND REACTIVITY OF MONOCLONAL ANTIBODIES AGAINST COLON CANCER BY IMMUNOPATHOLOGY IN NORMAL FETAL, NORMAL ADULT AND CANCER TISSUE IN HUMAN SPECIMENS

MONOCLONAL ANTIBODIES

| CANCER TISSUE | 85 | 28 | 6 | 29/36 | 29/15 | 479 | 15 | 174 | 86 | 70 | 152 | 307 | 218 | 29/26 | 68 |
|---|---|---|---|---|---|---|---|---|---|---|---|---|---|---|---|
| ASTROCYTOMA | 0 | 0 | | | 0 | 0 | 0 | 0 | 0 | | 0 | | | 0 | |
|  | 0 | 0 | | | 0 | 0 | 0 | 0 | 0 | | 0 | | | 0 | |
| LYMPHOMA | 0 | 0 | | | 0 | 0 | 0 | 0 | 0 | | 0 | | | 0 | |
|  | 0 | 0 | | | 0 | 0 | 0 | 0 | 0 | | 0 | | | 0 | |
| KIDNEY CANCER | 0 | ● | | | 0 | 0 | 0 | 0 | ● | | 0 | | | 0 | |
|  | 0 | ● | | | 0 | 0 | 0 | 0 | 0 | | ● | | | 0 | |
|  | 0 | 0 | | | 0 | 0 | 0 | 0 | ● | | ● | | | 0 | |
|  | 0 | 0 | | | 0 | 0 | 0 | 0 | 0 | | 0 | | | ● | |
|  | 0 | 0 | | | 0 | 0 | 0 | 0 | ● | | 0 | | | ● | |

43

TABLE III  REACTIVITY OF MOUSE MONOCLONAL ANIBODIES GENERATED
AGAINST COLORECTAL CANCERS.
SEROLOGICAL TEST WITH CULTURE HUMAN CELLS

| CELLS<br>Immunoglobulin subclass<br>Molecular weight | V-315<br>γ-3<br>gp 140 | A-33<br>γ-2b | K-314<br>γ-1<br>gp 170 | A-37<br>γ-1 | H-15<br>γ-1 | V-715<br>γ-1<br>gp 120 | M-70<br>μ<br>gp 31 | H-26<br>γ-2a<br>gp 29 |
|---|---|---|---|---|---|---|---|---|
| **ENTODERM ORIGIN TUMORS** | | | | | | | | |
| **COLON** | | | | | | | | |
| HT-29,SW-480,SW-403 | ● ○ ⊕ | ○ ○ ⊕ | ● ⊕ ● | ○ ● ● | ● ● ● | ● ⊕ ○ | ● ● ⊕ | ● ● ● |
| SW-48,CACO-2,SW-1116 | ⊕ ⊕ ○ | ○ ○ ⊕ | ● ● ● | ○ ○ ○ | ○ ● ● | ● ⊕ ⊕ | ○ ● ○ | ● ● ● |
| SK-CO-10,SK-CO-13 | ○ ○ | ○ ○ | ● ○ | ○ ● | ● ○ | ○ ⊕ | ● ● | ● ● |
| SW-1417,SW-1222,SK-CO-15 | ● ○ ⊕ | ○ ○ ○ | ● ● ⊕ | ○ ○ ○ | ● ● ⊕ | ● ○ ○ | ● ● ○ | ● ● ● |
| SW-620,SW-837,SK-CO-11 | ○ ⊕ ○ | ○ ⊕ ⊕ | ● ⊕ ○ | ○ ○ ○ | ● ○ ○ | ○ ⊕ ○ | ● ○ ⊕ | ● ● ○ |
| SW-1083,SK-CO-12,SK-CO-1 | ○ ○ ⊕ | ○ ○ ○ | ○ ○ ● | ○ ○ ● | ○ ⊕ ● | ○ ○ ○ | ● ● ⊕ | ● ● ● |
| **PANCREAS** | | | | | | | | |
| ASPC-1,CAPAN-1,CAPAN-2 | ○ ⊕ ○ | ● ○ ○ | ● ● ● | ● ● ● | ● ○ ⊕ | ○ ○ ○ | ● ● ⊕ | ● ● ● |
| **HEPATIC AND BILIARY** | | | | | | | | |
| SK-HEP-1,SK-CHL-2 | ○ ○ | ○ ○ | ○ ○ | ○ ○ | ● ● | ○ ○ | ● ○ | ● ● |
| **LUNG** | | | | | | | | |
| J-82,CALU-1,CALU-5 | ○ ○ ○ | ○ ○ ○ | ○ ● ○ | ○ ○ ○ | | ○ ● ○ | ○ ○ ○ | |
| CALU-6,SK-MES-1,SK-LU-1 | ○ ○ ○ | ○ ○ ○ | ● ● ● | ○ ○ ○ | | ○ ○ ⊕ | ○ ○ ○ | |
| SK-LC-1,-2,-4 | ○ ○ ○ | ○ ○ ○ | ● ○ ○ | ○ ○ ○ | ● | ● ● ○ | ○ ○ ○ | ● |
| SK-LC-5,-6,-9 | ○ ○ ○ | ○ ○ ○ | ● ○ ○ | ○ ○ ○ | ● ● ○ | ○ ● ○ | ○ ○ ○ | ● ○ ● |
| SK-LC-8,-10,-12 | ○ ○ ○ | ○ ○ ○ | ● ○ ○ | ○ ○ ○ | | ○ ○ ○ | ○ ○ ○ | |
| SK-LC-15,-16,-17 | ○ ○ ● | ○ ○ ○ | ○ ○ ○ | ○ ○ ○ | | ○ ○ ○ | ○ ○ ○ | |
| SK-LC-18,-19,-23 | ○ ○ ○ | ○ ○ ○ | ○ ○ ○ | ○ ○ ○ | | ○ ○ ○ | ○ ○ ○ | |
| SK-LC-24,-25,-28 | ○ ○ ○ | ○ ○ ○ | ○ ○ ○ | ○ ○ ○ | | ○ ○ ○ | ○ ○ ○ | |
| SK-LC-LL | ○ | ○ | ○ | ○ | ● | ○ | ○ | ● |
| **BLADDER** | | | | | | | | |
| 253-J,SW-780,TCCSUP | ○ ○ ○ | ○ ○ ○ | ○ ● ○ | ○ ○ ○ | ○ ○ ○ | ● ○ ○ | ● ● ○ | ○ ● ○ |
| 5637,VM-CUB-1,VM-CUB-2 | ○ ○ ○ | ○ ○ ○ | ○ ○ ○ | ○ ○ ○ | ○ ○ | ○ ○ ○ | ○ ○ ○ | ● ● |
| VM-CUB-3,575-A,RT-4 | ○ ○ ○ | ○ ○ ○ | ○ ○ ○ | ○ ○ ○ | ⊕ | ○ ○ ○ | ○ ○ ○ | ● |
| 639-V | ○ | ○ | ○ | ○ | ○ | ○ | ○ | ⊕ |
| **ECTODERM ORIGIN TUMORS** | | | | | | | | |
| **BREAST** | | | | | | | | |
| MDA-MB-361,MCF-7,CAMA | ○ ○ ○ | ○ ○ ○ | ○ ○ ○ | ○ ○ ○ | ○ ● ● | ○ ○ ○ | ○ ● ○ | ○ ● ○ |
| SK-BR-3,MDA-MB-157,ALAB | ○ ○ ○ | ○ ○ ○ | ○ ○ ○ | ○ ○ ○ | ● ○ ⊕ | ○ ○ ○ | ● ● ○ | ○ ○ ● |

VM-88,MeWo,SK-MEL-13
SK-MEL-23,-27,-28
SK-MEL-29,-33,-37
SK-MEL-42,-64,-73
SK-MEL-90,-129,-133
SK-MEL-176

ASTROCYTOMA
SK-MG-1,-2,-3
SK-MG-4,-9,10
SK-MG-12,-13,-14
SK-MG-16,MS,U-343
A-582

NEUROBLASTOMA
MC-NB-1,SMS-KHN,SKN-MC
SMS-SHN,SKN-BE

MESODERM ORIGIN TUMOR

RENAL CANCER
SK-RC-1,-2,-4
SK-RC-7,-9,-10
SK-RC-17,-18,-21
SK-RC-26A,-26B,28
SK-RC-29,-35,-37
SK-RC-39,-42,-44
SK-RC-45,-48

HEMATOPOIETIC TUMOR

B CELL
ARH 77 AG,ARA 10,DAUDI
SK-LY-16,-18,BALL-1
SK-DHL-2,SKO-007,RAJI
LICR LON,UC 729-6

T CELL
HPB ALL,T-45,MOLT-4
CCRF-HSB 2,CCRF-CEM,P-12

MONOCYTE
HL60,K-562,KG-1-G

NULL CELL
NALM-1,NALM-16,NKL 1
NKL 2,NALL 1

OTHER TUMOR

OVARIAN CANCER

| | | | | | | | |
|---|---|---|---|---|---|---|---|
| TURANECK,ROAC,A-7 | ⊖ O O | O O O | O O O | O O O | ● O O | ⊖ O O | ⊖ ● O | ● ● ● |
| SW-626 | O | O | O | O | ● | O | O | ● |

TERATOCARCINOMA

| | | | | | | | |
|---|---|---|---|---|---|---|---|
| 577M,TERA 1,833 K | O ● O | O O O | O O O | ● ⊖ O | O O O | O ⊖ ⊖ | O O O | O O O |

CHORIOCARCINOMA

| | | | | | | | |
|---|---|---|---|---|---|---|---|
| SWCC,MHCC | O O | O O | O O | O ⊖ | O O | ● O | O O | O O |

NORMAL CELLS

NORMAL FIBROBLAST

| | | | | | | | |
|---|---|---|---|---|---|---|---|
| #1,#2,#3 | O O O | O O O | O O O | O O O | O O O | O O O | O O O | O O O |
| #4,#5,#6 | O O O | O O O | O O O | O O O | O O O | O O O | O O O | O O O |
| #7,#8 | O O | O O | O O | O O | O O | O O | O O | O O |

NORMAL KIDNEY CELLS

| | | | | | | | |
|---|---|---|---|---|---|---|---|
| #1,#2, | O O | O O | O O | O O | O O | ⊖ ⊖ | O O | O O |

The symbols listed under the antibodies refer to the titer against the cell lines corresponding position in the left hand side of the table. The titer of the antibodies defined as the hightest dilution producing at least 50% rosetting in the MHA assay. ●, $1X10^{-6} - 1X10^{-3}$ ; ⊖, positive reaction but with over 50% rosetting at $10^{-3}$ dilution of antibody; ⊖, positive only with the absorption test; O, no reactivity at antibody dilution of $10^{-3}$.

REACTIVITY OF ANTIBODIES GENERATED AGAINST GASTROINTESTINAL
CANCERS: IMMUNOFLUORESCENCE TESTS WITH FROZEN SECTIONS OF NORMAL
HUMAN FETAL (F)* AND ADULT (A) TISSUES

| Normal Human Tissues | V-215 | | A-33 | | K-314 | | A-37 | | H-15 | | V-715 | | H-70 | | H-2 |
|---|---|---|---|---|---|---|---|---|---|---|---|---|---|---|---|
| | F | A | F | A | F | A | F | A | F | A | F | A | F | A | F |
| Colon | O | O | O | O | ● | ● | O | O | ● | ● | O | O | ● | ● | ● |
| Small intestine | O | O | O | O | ● | ● | O | O | O | ● | O | O | ● | ◉ | ◉ |
| Stomach | O | O | O | O | ● | ● | O | O | O | O | O | O | ● | ◉ | O |
| Esophagus | O | O | O | O | O | O | O | O | O | O | O | O | O | O | ◉ |
| Pancreas-Endocrine | O | O | O | O | O | O | O | O | O | O | O | O | O | O | O |
|    -Exocrine | O | O | O | O | ● | ● | O | O | ● | ● | O | O | ◉ | ◉ | ● |
| Liver-Hepatocytes | O | O | O | O | O | O | O | O | O | O | O | O | ◉ | ◉ | ● |
|   -Biliary epithelium | O | O | O | O | O | O | O | O | O | O | O | O | O | O | ● |
| Lung-Bronchial epithelium | ◉ | ◉ | O | O | ● | ● | O | O | ◉ | ◉ | O | O | ● | ● | ● |
|   -Pneumocytes | O | O | O | O | O | O | O | O | O | O | O | O | O | O | O |
| Urothelium | O | O | O | O | O | O | O | O | O | O | O | O | O | O | ● |
| Prostate | O | O | O | O | O | O | O | O | O | O | O | O | O | O | O |
| Kidney-Glomerulus | O | O | O | O | O | O | O | O | O | O | O | O | O | O | O |
|   -Proximal Tubules | O | O | O | O | ● | ● | ◉ | ◉ | O | O | ● | ● | ● | ● | O |
|   -Henle's Loop | O | O | O | O | ◉ | ◉ | O | O | O | O | O | O | O | O | O |
|   -Distal Tubules | O | O | O | O | O | O | O | O | O | O | O | O | O | O | ● |
|   -Collecting Tubules | O | O | O | O | O | O | O | O | O | O | O | O | O | O | ● |
| Testis-Germ Cells | O | O | O | O | O | O | O | O | O | O | O | O | O | O | O |
|   -Endocrine cells | O | O | O | O | O | O | O | O | O | O | O | O | O | O | O |
| Ovary | O | O | O | O | O | O | O | O | O | O | O | O | O | O | O |
| Placenta-Syncytrophoblast | O | O | O | O | O | O | O | O | O | O | O | O | O | O | O |
|    -Cytotrophoblast | O | O | O | O | O | O | O | O | O | O | O | O | O | O | O |
| Uterus-Endometrium | ◉ | ◉ | O | O | ● | ● | O | O | O | O | O | O | ● | ● | ● |
|   -Myometrium | O | O | O | O | O | O | O | O | O | O | O | O | O | O | O |
| Cervix-Endocervix | O | O | O | O | O | O | O | O | O | O | O | O | O | O | ● |
|   -Exocervix | O | O | O | O | O | O | O | O | O | O | O | O | O | O | O |
| Breast-Duct cells | O | O | O | O | O | O | O | O | O | O | O | O | O | O | O |
|   -Acinar cells | O | O | O | O | O | O | O | O | O | O | O | O | O | O | O |
| Adreanl | O | O | O | O | O | O | O | O | O | O | O | O | O | O | O |
| Skin-Epidermis | O | O | O | O | O | O | O | O | O | O | O | O | O | O | ◉ |
|   -Adnexa | O | O | O | O | O | O | O | O | O | O | ◉ | ◉ | ◉ | ◉ | ● |
|   -Melanocytes | O | O | O | O | O | O | O | O | O | O | O | O | O | O | O |
| Brain-Neurons | O | O | O | O | O | O | O | O | O | O | O | O | O | O | O |
|   -Glial cells | O | O | O | O | O | O | O | O | O | O | O | O | O | O | O |
|   -Dendrites | O | O | O | O | O | O | O | O | O | O | O | O | O | O | O |
| Thyroid | O | O | O | O | O | O | O | O | O | O | O | O | O | O | O |
| Spleen-White Pulp | O | O | O | O | O | O | O | O | O | O | O | O | O | O | O |
|   -Red Pulp | O | O | O | O | O | O | O | O | O | O | O | O | O | O | O |
| Lymph Nodes | O | O | O | O | O | O | O | O | O | O | O | O | O | O | O |
| Thymus | O | O | O | O | O | O | O | O | O | O | O | O | O | O | O |
| Heart | O | O | O | O | O | O | O | O | O | O | O | O | O | O | O |
| Muscle | O | O | O | O | O | O | O | O | O | O | O | O | O | O | O |
| Endothelial cells | O | O | O | O | O | O | O | O | O | O | O | O | O | O | O |
| Fibroblasts | O | O | O | O | O | O | O | O | O | O | O | O | O | O | O |
| Cartilage | O | O | O | O | O | O | O | O | O | O | O | O | O | O | O |
| Interstitial Matrix | O | O | O | O | O | O | O | O | O | O | O | O | O | O | O |
| Secretions | ◉ | ◉ | O | O | ● | ● | O | O | ◉ | ● | O | O | ● | ◉ | ● |

Reactivity of monoclonal antibodies with tissue sections is symbolized as follc
O, no immunofluorescence; ●, immunofluorescence, ◉, heterogenous pattern of
immunofluorescence.
*Fetal tissues were obtained from a 14 weeks old fetus.

48 4/12/85

# TABLE V   REACTIVITY OF MONOCLONAL ANTIBODIES WITH THE TUMOR AND NORMAL ADJACENT FROZEN TISSUE SECTIONS OF CANCER PATIENT

| Location | Patient | Monoclonal antibodies | | | | | |
| --- | --- | --- | --- | --- | --- | --- | --- |
| | | V-215 | A-33 | K-313 | H-15 | V-715 | H-26 |
| Rectal colon | AR | − | + | − | − | − | + |
| + | AY | − | + | ● | + | − | + |
| Sigmoid colon | BR | − | + | + | ● | − | + |
| | BT | − | + | + | + | − | + |
| | CH | ● | + | + | ● | ● | + |
| | CR | − | + | + | + | − | + |
| | DE | − | + | ● | − | − | + |
| | DN | ● | + | ● | ● | ● | + |
| | FA | − | + | ● | − | ● | + |
| | FU | − | + | ● | − | − | + |
| | GA | − | + | ● | − | + | + |
| | GF | − | + | + | + | − | + |
| | GY | ● | + | ● | + | − | + |
| | HT | ● | + | ● | ● | − | + |
| | MK | − | + | + | + | − | + |
| | MS | − | + | ● | + | − | + |
| | NC | − | + | + | − | − | + |
| | ND | − | − | − | − | − | + |
| | OT | − | + | + | ● | ● | + |
| | PP | − | − | + | − | − | + |
| | RD | − | + | ● | + | − | + |
| | RT | − | + | ● | + | − | + |
| | RV | − | + | + | − | − | + |
| | VC | − | + | + | − | − | + |
| Left colon | BW | − | + | + | ● | − | + |
| | HL | ● | + | ● | + | − | + |
| | MT | − | + | + | − | ● | + |
| | SM | − | − | + | − | − | + |
| | ST | − | + | + | − | − | + |
| Right colon | BA | ● | + | + | − | − | + |
| | BG | − | + | + | − | − | + |
| | FS | − | + | ● | ● | − | + |
| | GA | − | + | + | + | − | + |
| | HA | − | + | + | − | − | + |
| | HU | + | + | + | − | − | + |
| | KP | − | − | ● | ● | − | + |
| | SH | − | − | − | − | − | + |

What is Claimed:

1. Monoclonal antibodies characterized by immunological binding to human gastro-intestinal cell antigens and wherein said monoclonal antibody is selected from the group consisting of V-215, K-314, V-715, AS-33, and AS-37.

2. Monoclonal-antibody-producing-hybridoma cell line formed by fusing a myeloma cell line and spleen cells derived from a mammal immunized with established culture cell lines of human gastrointestinal cell carcinomas, pancreatic tumor cell lines, wherein the monoclonal antibody is selected from the group consisting of monoclonal antibody V-215, K-314, V-715, AS-33 and AS-37.

3. Panel of monoclonal antibodies for the diagnosis of human gastrointestinal cancer wherein the panel consists of two or more different monoclonal antibodies selected from the group consisting of V-215, K-314, V-715, AS-33, and AS-37.

4. Panel of claim 3 wherein the human gastrointestinal cancer diagnosed is human colon cancer.

5. Panel of monoclonal antibodies for the diagnosis of human gastrointestinal cancer wherein the panel consists of two or more different monoclonal antibodies selected from the group consisting of AS33, AS37, V-214, V-715, CLH70, CLK314, CLT86, CLT307, HT 29-15, and HT 29-26.

6. Panel of claim 5 wherein the human gastrointestinal cancer diagnosed is human colon cancer.

7. Method for differentiating normal and abnormal gastrointestinal cells which comprises contacting a human gastrointestinal specimen containing gastrointestinal cellular material with two or more of the monoclonal antibodies of from the group consisting of AS-33, AS-37, V-215, V-715, CLH70, CLK 314, CLT86, CLT307, HT29-15 and HT 29-26 and detecting the presence or absence of immune complex formation with two or more of said monoclonal antibodies indicating the presence or absence of abnormality in the gastrointestinal specimen.

8.  Method of Claim 7 wherein the abnormality is gastro-
    intestinal cancer.

9.  Method of Claim 7 wherein the abnormality is colon
    cancer.

10. Method of Claim 7 wherein the specimen is contacted
    singly, serially or in combination with each of hte
    panel monoclonal antibodies.